# EUROPÄISCHE PATENTSCHRIFT

(11) **EP 2 918 298 B1**
(45) Veröffentlichungstag und Bekanntmachung des Hinweises auf die Patenterteilung: **19.05.2021**
(21) Anmeldenummer: 14159635.3
(22) Anmeldetag: 13.03.2014
(51) Int. Cl.: A61M 5/20, A61M 5/315

(54) **Verbesserter Dosiermechanismus für eine Injektionsvorrichtung zum Verabreichen eines Produkts**
Improved dosing mechanism for an injection device for administering a product
Mécanisme de dosage amélioré pour un dispositif d'injection pour l'administration d'un produit

(43) Veröffentlichungstag der Anmeldung: 16.09.2015
(73) Patentinhaber: Ypsomed AG, 3401 Burgdorf (CH)
(72) Erfinder: Streit, Ursina, 3322 Schönbühl (CH); Schenker, Susanne, 4900 Langenthal (CH); Hostettler, Patrick, 3415 Hasle (CH)
(74) Vertreter: Kleiner, Stefan

(56) Entgegenhaltungen:
- EP-A1- 0 554 995
- WO-A1-2013/170392
- WO-A2-2004/078226

## Beschreibung

Die Erfindung betrifft einen Dosiermechanismus für eine Injektionsvorrichtung, mit der ein vorzugsweise flüssiges Produkt, insbesondere Medikament, verabreichbar oder ausschüttbar ist. Insbesondere betrifft die Erfindung auch eine Injektionsvorrichtung, die einen solchen Dosiermechanismus aufweist. Mittels des Dosiermechanismus ist eine zu verabreichende Dosis einstellbar. Der Dosiermechanismus kann Teil eines Antriebsmechanismus sein, so dass es sich bevorzugt auch um einen Dosier- und Antriebsmechanismus handeln kann. Der Mechanismus kann das Einstellen einer Dosis verhindern, welche zum Beispiel die Menge eines zu verabreichenden Produkts in einem Produktbehälter der Injektionsvorrichtung übersteigt.

Der Begriff "Medikament" umfasst hier jede fließfähige medizinische Formulierung, welche geeignet ist zur kontrollierten Verabreichung durch ein Mittel, wie z. B. eine Kanüle oder Hohlnadel, hindurch, beispielsweise umfassend eine Flüssigkeit, eine Lösung, ein Gel oder eine feine Suspension, welche(s) einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form aber auch Polysaccharide, Vaccine, DNS oder RNS oder Oglionukleotide, Antikörper oder Teile von Antikörpern sowie geeignete Basis-, Hilfs- und Trägerstoffe

Die Erfindung bezieht sich insbesondere auf einen Dosiermechanismus, welcher das Einstellen einer zu verabreichenden Dosis über einen vorgegebenen Wert hinaus verhindert. Somit kann das Einstellen einer Dosis bereits dann verhindert werden, wenn in dem Produktbehälter noch ausreichende Mengen für weitere Injektionen vorhanden wäre. Hierdurch kann vorteilhaft erreicht werden, dass eine gewünschte abgebbare Produktmenge durch das Gerät und nicht durch die in dem Produktbehälter enthaltene Gesamtmenge vorgegeben wird.

Aus dem Stand der Technik ist eine Vielzahl von Dosiermechanismen bekannt, welche die Einstellung einer Dosis verhindern, welche die Medikamentenmenge in einem Reservoir einer Injektionsvorrichtung übersteigt.

Mit aus dem Stand der Technik bekannten Injektionsvorrichtungen können Produktdosen mit einem Dosiermechanismus eingestellt und anschließend aus dem Produktbehälter ausgeschüttet werden. Es kann der Fall auftreten, dass mit einem Dosiseinstellelement eine größere Dosis eingestellt wurde, als aus dem Produktbehälter ausschüttbar ist, weil der Produktbehälter eine geringere Produktmenge enthält als die Dosis die eingestellt wurde. Dies kann dazu führen, dass weniger Produkt ausgeschüttet wird, als eingestellt wurde, was je nach Abweichung zu mehr oder weniger Problemen für den Patienten führen kann.

Beispielsweise ist in der Offenlegungsschrift WO 2004/078226 A2 ein Antriebsmechanismus für Medikamentenverabreichungsgeräte beschrieben. Dieser Antriebsmechanismus enthält ein Gehäuse, eine Dosiseinstellhülse und eine zweiteilige Kolbenstange. In einer Ausführungsform läuft eine Treiberhülse abwärts entlang einem inneren Teil der Kolbenstange, wenn eine Dosis gewählt wird. Die zurückgelegte Strecke entspricht dabei dem für die Dosis nötigen Ausschütthub des Kolbens. Wenn eine nachfolgende Dosis gewählt wird läuft die Treiberhülse weiter entlang der Kolbenstange. Die Position der Treiberhülse entspricht also der noch in der Karpule enthaltenen Medikamentenmenge. Wenn die Treiberhülse dann das Ende eines Gewindegangs am inneren Teil der Kolbenstange erreicht und dadurch nicht weiter rotieren kann korrespondiert das mit einer leeren Karpule.

Ein weiteres Beispiel ist in der Offenlegungsschrift US 6,582,404 B1 beschreiben, welche einen Begrenzungsmechanismus für Medikamentenverabreichungsgeräte zeigt, welcher das Einstellen einer Dosis verhindert, welche den in der Karpule verbliebenen Rest überschreitet. Die Verabreichungseinrichtung weist ein Dosiseinstellglied auf, welches beim Setzen einer Dosis durch Drehung relativ zu einem Treiber von einem festen Anschlag wegbewegt wird. Das Dosiseinstellglied ist dabei so mit dem Treiber verbunden, dass jenes in einer Richtung gedreht werden kann ohne diesen mitzunehmen. Die Dosis wird verabreicht, indem das Dosiseinstellglied zurückgedreht wird und dabei den Treiber mitnimmt. Der drehende Treiber verursacht eine Ausschüttbewegung der Kolbenstange. Der Treiber ist mit einer Spur versehen, deren Länge mit der nominal in der Karpule enthaltenen Medikamentenmenge korrespondiert. in dieser Spur läuft ein Spurfolger, welcher mit dem Dosiseinstellglied verbunden ist. Jedes Mal, wenn eine Dosis gewählt wird, bewegt sich der Spurfolger in der Spur weiter. Wenn der Spurfolger das Ende der Spur erreicht, kann das Dosiseinstellglied nicht weiter gedreht werden und eine Dosiseinstellung über den in der Karpule noch vorhandenen Rest hinaus wird verhindert.

Ein weiteres Beispiel eines solchen Geräts ist in der EP 0 554 996 A1 beschrieben und zeigt eine Injektionsvorrichtung um Flüssigkeiten wie Insulin in Körpergewebe zu verabreichen. Diese Injektionsvorrichtung enthält einen Dosissetzmechanismus welcher einen Einerring und einen Zehnerring aufweist. Ein Übertragungsglied ist vorgesehenen, um selektiv den Einermit dem Zehnerring zu koppeln, damit diese nur in gewählten Abschnitten während dem Dosiseinstellen miteinander drehen. Die eingestellte Dosis wird mittels Ziffern auf den Ringen angezeigt. Weiter weist die Injektionsvorrichtung einen Dosisbegrenzungsmechanismus auf, welcher die Bewegung einer Führungsspindel für die vorgesehene Kolbenbewegung in der Karpule begrenzt, wobei Abragungen im Stößel an das Ende von Nuten entlang der Führungsspindel gelangen und eine weitere Bewegung verhindern. Der Dosisbegrenzungsmechanismus ist separat vom Dosissetzmechanismus vorgesehen.

Schließlich zeigt die WO 2006/086983 A1 ein Beispiel einer Dosiseinstelleinrichtung für Selbstinjektionsgeräte mit einem Dosisbegrenzungsmechanismus, welcher zwei drehende Teile aufweist, wobei das erste Teil während dem Einstellen einer Dosis kontinuierlich dreht und das zweite Teil jeweils nach Erreichen einer bestimmten Drehposition über eine selektive Kupplungseinrichtung abschnittsweise mitdreht. Dadurch wird erreicht, dass das zweite Teil diskontinuierlich über einen kleineren Winkel dreht als das erste Teil. Die Drehung des zweiten Teils wird sodann begrenzt durch einen gehäusefesten Anschlag, welcher eine Dosiseinstellung über den in der Karpule noch vorhanden Rest hinaus verhindert.

Aus der WO 2010/149209 A1 ist eine Injektionsvorrichtung bekannt, welche ein Dosiseinstellelement und ein damit gekoppeltes Element, welches bei einer Dosiseinstellung relativ zu einem anderen, zweiten Element drehbar und bei einer Dosisausschüttung relativ zu dem zweiten Element drehfest ist. Das erste Element und das zweite Element sind über ein Koppelglied, welches zum Beispiel kugel-, ring-, mutter- oder segmentförmig sein kann, gekoppelt. Ferner ist ein Stoppanschlag vorgesehen, wobei bei einer Dosiseinstellung das Koppelglied eine Bewegung zu einer Stoppposition hin ausführt, wobei das Koppelglied in der Stoppposition die Einstellung einer Dosis verhindert.

Die WO 2013/170392 A1 beschreibt einen Dosiermechanismus, welche das Einstellen einer zu verabreichenden Dosis über einen vorgegebenen Wert hinaus zuverlässig, einfach und platzsparend verhindert. Hierfür können ein erstes und ein zweites Stoppmittel während der Dosiseinstellung jeweils eine Bahnkurve durchlaufen. Die vom ersten und zweiten Stoppmittel beschriebenen Bahnkurven sind in sich geschlossen und können vom ersten Stoppmittel und/oder vom zweiten Stoppmittel mehrmals durchlaufen werden, bis die Stoppmittel an der Stoppposition in einen gegenseitigen Anschlag gelangen, wodurch bei der Dosierbewegung eine Blockierung der Bewegung der Begrenzungsmittel relativ zueinander bewirkt wird.

Der vorliegenden Erfindung liegt die Aufgabe zugrunde, einen Dosiermechanismus zum Einstellen einer Dosis für ein Injektions- oder Infusionsgerät zur Verabreichung eines Produkts zu schaffen, welcher das Einstellen einer zu verabreichenden Dosis über einen vorgegebenen Wert zuverlässig, einfach und noch platzsparender verhindert und möglichst stabil aufgebaut ist.

Diese Aufgabe wird durch den Gegenstand der unabhängigen Ansprüche gelöst. Vorteilhafte Weiterentwicklungen ergeben sich aus den abhängigen Ansprüchen, der Beschreibung und den Figuren.

Die Erfindung wird von einem Dosiermechanismus für eine Injektionsvorrichtung aus, wobei der zentrale Gedanke ist, dass ein mit einem Dosiseinstellelement gekoppeltes erstes Element während einer Dosiseinstellung relativ zu einem anderen, zweiten Element um eine Drehachse in eine erste Drehrichtung drehbar und während einer Dosisausschüttung relativ zu dem zweiten Element drehfest ist, wobei das erste Element und das zweite Element über ein Stoppelement gekoppelt sind. Hierdurch wird ein Zählwerk gebildet, das nur die Dosen erfasst, die während der Dosiseinstellung eingestellt werden. Da das erste Element und das zweite Element relativ zueinander verdrehfest sind, wenn die eingestellte Dosis ausgeschüttet wird, kann sich der Dosiermechanismus den Absolutwert, d. h. die Summe aller eingestellten ausgeschütteten Dosen merken.

Das Dosiseinstellelement kann mit dem ersten Element mittelbar oder unmittelbar gekoppelt werden. Bevorzugt ist das Dosiseinstellelement mit dem ersten Element beim Einstellen der Dosis drehfest gekoppelt. Die Kopplung kann beispielsweise bei der Ausschüttung der eingestellten Produktdosis gelöst sein, insbesondere über eine Kupplung. Alternativ kann das Dosiseinstellelement permanent mit dem ersten Element drehfest gekoppelt sein, insbesondere wenn das Dosiseinstellelement eine Dosisanzeigehülse ist oder aufweist oder zumindest mit einer solchen gekoppelt ist.

Die Funktion, dass das erste Element relativ zu dem zweiten Element während einer Dosiseinstellung drehbar ist und während der Dosisausschüttung drehfest ist, wird mittels einer Kupplung realisiert. Diese Kupplung kann während der Dosiseinstellung ausgekuppelt oder geöffnet sein, wobei sie während der Ausschüttung eingekuppelt oder geschlossen sein kann. Die Kupplung kann zum Beispiel eine erste Kupplungsstruktur aufweisen, die mit dem ersten Element verdrehgesichert verbunden ist, insbesondere von dem ersten Element gebildet wird, und eine weitere, zweite Kupplungsstruktur, die mit dem zweiten Element drehfest gekoppelt ist, insbesondere von dem zweiten Element gebildet wird, aufweisen. Die erste Kupplungsstruktur und die zweite Kupplungsstruktur greifen formschlüssig ineinander, wenn die Kupplung eingekuppelt ist, so dass die erste Kupplungsstruktur relativ zu der zweiten Kupplungsstruktur verdrehgesichert ist. Wenn die Kupplung ausgekuppelt ist, sind die beiden Kupplungsstrukturen relativ zueinander verdrehbar und vorzugsweise aus dem formschlüssigen Eingriff. Zum Beispiel kann ein Betätigungsglied, insbesondere Betätigungskopf vorgesehen sein, der zwischen einer unbetätigten Position und einer betätigten Position hin und her bewegbar, wie z.B. verschiebbar ist, wobei das Betätigungsglied so mit der Kupplung verbunden ist, dass die Kupplung in der unbetätigten Position ausgekuppelt und in der betätigten Position eingekuppelt ist. Insbesondere wird die ausgekuppelte Kupplung durch Betätigen des Betätigungsglieds, d. h. durch Bewegen oder Verschieben des Betätigungsglieds aus seiner unbetätigten Position in die betätigte Position, eingekuppelt. Beispielsweise können die beiden Kupplungsstrukturen, der Kupplung relativ zueinander zum Beispiel entlang der Drehachse verschoben werden, wenn das Betätigungsglied betätigt oder losgelassen wird. Zum Beispiel kann eine Feder vorgesehen sein, welche die Kupplung in ihrem ausgekuppelten Schaltzustand und/oder das Betätigungsglied in seiner unbetätigten Position hält. Die Feder ist durch Einkuppeln der Kupplung spannbar. Wenn das Betätigungsglied betätigt wird, kann die Feder gespannt werden, wobei die gespannte Feder des Betätigungsglieds in eine unbetätigte Position zurücksetzt und/oder die Kupplung auskuppelt, wenn das Betätigungsglied aus der betätigten Position in die unbetätigte Position bewegt wird, insbesondere vom Verwender des Dosiermechanismus losgelassen wird.

Das Betätigungsglied kann zum Beispiel ein Betätigungsknopf sein, der am proximalen Ende, welches dem distalen Ende, an dem eine Injektionsnadel angeordnet werden kann, gegenüberliegt, der Vorrichtung angeordnet ist oder das proximale Ende bildet. Das Betätigungsglied kann mit einem Finger, insbesondere dem Daumen der Hand des Verwenders, welche ein Gehäuse des Dosiermechanismus oder der Injektionsvorrichtung, die den Dosiermechanismus aufweist, umgreift, gedrückt werden, d. h. betätigt bzw. aus der unbetätigten Position in die betätigte Position verschoben werden. Durch Loslassen des Betätigungsglieds kann die Feder das Betätigungsglied in die unbetätigte Position zurücksetzen.

Das erste, vorzugsweise hülsenförmige Element und das zweite, vorzugsweise hülsenförmige Element sind über ein insbesondere hülsenförmiges Stoppelement, welches einen Stoppgegenanschlag bildet, gekoppelt. Insbesondere können das erste Element und das zweite Element jeweils in einem Kontakt mit dem hülsenförmigen Stoppelement sein.

Besonders bevorzugt kann das hülsenförmige Stoppelement eines aus erstem Element und zweitem Element umgeben, wobei z.B. das andere aus erstem und zweitem Element das hülsenförmige Stoppelement umgeben kann. Zum Beispiel kann zwischen dem ersten Element und dem zweiten Element ein Ringspalt gebildet sein, in dem das hülsenförmige Stoppelement angeordnet ist. Dadurch dass das hülsenförmige Stoppelement das erste Element oder das zweite Element umgibt, wird eine besonders stabile Ausführung erreicht. Insbesondere kann das hülsenförmige Stoppelement eine Kolbenstange umgeben oder einen Durchgang für die Kolbenstange bilden. Die Kolbenstange wird zum Ausschütten der Produktdosis in eine Ausschüttrichtung bewegt. Hierdurch lässt sich eine kompakte Bauweise erzielen, insbesondere die Baulänge des Dosiermechanismus verringern oder gering halten.

Der Dosiermechanismus weist einen Stoppanschlag auf, wobei der Stoppgegenanschlag während der Drehung des ersten Elements in die erste Drehrichtung eine Bewegung zu einer Stoppposition hin ausführt, wobei in der Stoppposition der Stoppgegenanschlag an dem Stoppanschlag anschlägt und die Drehung des ersten Elements relativ zu dem zweiten Element in die erste Drehrichtung verhindert. Hierdurch wird verhindert, dass das erste Element weiter in die erste Drehrichtung, d. h. die Drehrichtung bewegt wird, die eine Erhöhung der auszuschüttenden Dosis bewirken würde. Der Stoppgegenanschlag kann eine Stoppgegenanschlagfläche und der Stoppanschlag kann eine Stoppanschlagfläche aufweisen, wobei diese Flächen in einem Kontakt sind, wenn der Stoppgegenanschlag an dem Stoppanschlag anschlägt.

Beispielsweise kann das Dosiseinstellelement, insbesondere von dem Verwender der Vorrichtung für ein Aufdosieren, d. h. für ein Erhöhen der auszuschüttenden Dosis, in die erste Drehrichtung und zum Abdosieren, welches auch als Dosiskorrektur bezeichnet werden, in eine zweite, der ersten Drehrichtung entgegengesetzte Drehrichtung gedreht werden, insbesondere in Bezug auf das zweite Element und/oder ein Gehäuse, in welchem das erste Element, das zweite Element und das Stoppelement angeordnet sein können. Das Gehäuse kann zum Beispiel hülsenförmig sein, insbesondere zylindrisch und die ersten und zweiten Elemente sowie den Stoppgegenanschlag umgeben oder aufnehmen.

Zum Beispiel kann das Dosiseinstellelement relativ zu dem Gehäuse in die erste und/oder zweite Drehrichtung gedreht werden.

In einem ersten Aspekt kann das hülsenförmige Stoppelement um eine Nebendrehachse, die parallel versetzt oder winkelversetzt zur Hauptdrehachse, um welche das erste Element relativ zu dem zweiten Element drehbar ist, drehbar sein. Die Nebendrehachse ist die Rotationsachse des hülsenförmigen Stoppelements. Die Hauptdrehachse ist die Rotationsachse des ersten Elements. Insbesondere kann die Hauptdrehachse der Mittelachse des Gehäuses, des ersten Elements, des zweiten Elements und ggf. der Kolbenstange entsprechen.

Dass die Nebendrehachse parallel versetzt zur Hauptdrehachse angeordnet ist, bedeutet, dass die Nebendrehachse exzentrisch zur Hauptdrehachse angeordnet ist. Dass die Nebendrehachse winkelversetzt zur Hauptdrehachse angeordnet ist, bedeutet, dass die Nebendrehachse in einem Winkel größer 0°, insbesondere in einem spitzen Winkel, d. h. einem Winkel, größer 0° kleiner 90° zueinander angeordnet sind. Die Hauptdrehachse und die Nebendrehachse können einander schneiden oder windschief zueinander angeordnet sein. Beispielsweise kann die Nebendrehachse alternativ rechtwinklig zur Hauptdrehachse angeordnet sein, insbesondere die Hauptdrehachse schneiden oder windschief zur Hauptdrehachse angeordnet sein. Windschief bedeutet, dass die Hauptdrehachse und die Nebendrehachse einander nicht schneiden und nicht parallel zueinander angeordnet sind.

Dadurch dass die Nebendrehachse parallel versetzt oder winkelversetzt zur Hauptdrehachse angeordnet ist, kann das Stoppelelement eines aus erstem Element oder zweitem Element nur an einer Stelle seines Umfangs, insbesondere in einem Eingriffsbereich, berühren. Der übrige, insbesondere größte Teil des Umfangs des Stoppelements berührt das erste oder das zweite Element nicht. Durch diese Anordnung wird vorteilhaft erreicht, dass der Umfang, den das Stoppelement an der einen Stelle berührt, anders, insbesondere größer oder kleiner als der Umfang des Stoppelements ist. Hierdurch wird vorteilhaft erreicht, dass sich das Stoppelement und das erste oder zweite Element, welches das Stoppelement an der einen Stelle berührt, sich mit der gleichen Umfangsgeschwindigkeit, aber mit unterschiedlichen Winkelgeschwindigkeiten drehen. Dadurch wird in einer ähnlichen Weise wie bei der WO 2013/170392 A1 bewirkt, dass ein Punkt, insbesondere eine Schaltstruktur, die von dem Element, welches das Stoppelement an der einen Stelle berührt, gebildet wird, und ein Punkt, insbesondere eine Schaltgegenstruktur, die von dem Stoppelement gebildet wird, während der Drehung des ersten Elements relativ zu dem zweiten Element jeweils eine Bahnkurve so beschreiben, dass sich die beiden Bahnkurven an mindestens einer Stelle schneiden oder so nahe kommen, dass die Punkte aneinander so nahe kommen, dass sie in einem gegenseitigen Anschlag gelangen. Dieser Anschlag bewirkt zum Beispiel, dass eine Drehung des ersten Elements relativ zu dem zweiten Element in die erste Drehrichtung blockiert wird oder dass der Stoppgegenanschlag ausgelenkt wird, insbesondere quer zur Umfangsrichtung.

Die vom ersten und zweiten Punkt beschriebenen Bahnkurven können in sich geschlossen, insbesondere kreisförmig sein und vom ersten Punkt, vom zweiten Punkt oder von beiden Punkten, vorzugsweise mehrmals durchlaufen werden, bis die Punkte in den gegenseitigen Anschlag gelangen.

Beispielsweise kann das Stoppelement eine über seinen Umfang konzentrisch um die Nebendrehachse angeordnete erste Eingriffsstruktur, insbesondere eine Verzahnung aufweisen, und eines aus erstem Element und zweitem Element eine über seinen Umfang und konzentrisch um die Hauptdrehachse angeordnete zweite Eingriffsstruktur, insbesondere eine zweite Verzahnung, aufweisen, wobei die erste Eingriffsstruktur und die zweite Eingriffsstruktur an einer Stelle des Umfangs, d. h. im Eingriffsbereich, formschlüssig ineinandergreifen, insbesondere kämen. Außerhalb des Eingriffsbereichs, d. h. über den größeren Bereich des Umfangs des Stoppelements greifen die erste Eingriffsstruktur und die zweite Eingriffsstruktur nicht ineinander.

Sofern die erste Eingriffsstruktur und die zweite Eingriffsstruktur jeweils eine über den Umfang angeordnete Verzahnung ist, können die Wälzkreise dieser Verzahnungen vorzugsweise unterschiedlich groß sein.

Insbesondere kann das Stoppelement exzentrisch zu der Hauptdrehachse oder/und konzentrisch um die Nebendrehachse an dem aus erstem Element und zweitem Element gelagert, insbesondere gleitgelagert sein, welches nicht mit der oben genannten Eingriffsstruktur in einem Eingriff mit dem Stoppelement ist.

Das Stoppelement kann zum Beispiel eine erste Gleitfläche und das andere aus erstem Element und zweitem Element kann eine zweite Gleitfläche aufweisen, wobei mindestens eine aus erster Gleitfläche und zweiter Gleitfläche, vorzugsweise beide Gleitflächen, konzentrisch um die Nebendrehachse angeordnet ist. Die erste Gleitfläche und die zweite Gleitfläche können während der Drehung des ersten Elements drehend aneinander abgleiten. Die erste Gleitfläche kann sich zumindest teilweise, vorzugsweise mindestens über den größten Teil des Umfangs oder vollständig über den Umfang des Stoppelements erstrecken. Die zweite Gleitfläche kann sich zumindest teilweise, vorzugsweise mindestens über den größten Teil des Umfangs oder vollständig über den Umfang des ersten oder zweiten Elements erstrecken. Das Element, welches die zweite Gleitfläche aufweist, kann zum Beispiel hülsenförmig sein, wobei es mindestens einen Abschnitt, der sich konzentrisch um die Hauptdrehachse erstreckt, und einen Abschnitt, nämlich die Gleitfläche, der sich konzentrisch um die Nebendrehachse erstreckt, aufweisen kann.

Der Stoppanschlag kann von dem ersten Element oder dem zweiten Element gebildet sein. Zum Beispiel kann der Stoppanschlag von einer Stufe gebildet werden, die an dem Innen- oder Außenumfang gebildet ist, welcher die zweite Gleitfläche bildet. Alternativ kann der Stoppanschlag von einem Vorsprung gebildet werden, wobei zwischen dem Vorsprung und der zweiten Gleitfläche ein Spalt gebildet sein kann, in dem zum Beispiel das Stoppelement oder ein Teil des Stoppelements angeordnet sein kann.

In bevorzugten Ausführungen kann der Stoppgegenanschlag quer zur Umfangsrichtung auslenkbar, insbesondere verschiebbar oder vorzugsweise elastisch auslenkbar angeordnet, insbesondere an dem Stoppelement gebildet sein. D. h., dass der Stoppgegenanschlag in etwa radial nach außen oder radial nach innen, d. h. von der Nebendrehachse weg oder zu der Drehachse hin auslenkbar ist.

In weiteren Ausführungen kann das Stoppelement, einschließlich seines zum Beispiel starr daran gebildeten Stoppgegenanschlags, und/oder die Nebendrehachse um eine Kippachse, die quer, wie z.B. in etwa senkrecht, auf die Hauptdrehachse und die Nebendrehachse steht, in eine ausgelenkte Position verkippt werden, in der die Nebendrehachse winkelversetzt zur Hauptdrehachse angeordnet ist, wodurch der Stoppgegenanschlag dem Stoppanschlag in Umfangsrichtung gegenüberliegt. Das Verkippen kann dadurch erreicht werden, dass die Schaltstruktur und die Schaltgegenstruktur aneinander anschlagen. In der unausgelenkten Position, aus der das Stoppelement verkippt wird, kann die Nebendrehachse z.B. parallel versetzt zur Hauptdrehachse sein oder frei um die Hauptdrehachse kippbar sein. Wenn die Schaltstruktur und Schaltgegenstruktur aneinander anschlagen, wird das Verkippen des Stoppelements und der Nebendrehachse erzwungen. Bevorzugt sind die erste Eingriffsstruktur des Stoppelements und die Schaltgegenstruktur entlang der Nebendrehachse voneinander beabstandet. Hierdurch wird bewirkt, dass ein Kippmoment um die Kippachse erzeugt wird, wenn die Schaltstruktur und die Schaltgegenstruktur aneinander anschlagen.

Eines aus erstem Element und zweitem Element kann allgemein eine Schaltstruktur aufweisen, die insbesondere den Punkt bildet oder der Punkt ist, welcher die Bahnkurve zum Beispiel mehrfach durchlaufen kann. Die Schaltstruktur kann den Stoppgegenanschlag, an dem insbesondere eine Schaltgegenstruktur gebildet ist, während der Drehung des ersten Elements quer zur Umfangsrichtung, d. h. z.B. radial nach innen oder außen, auslenken, insbesondere elastisch oder verschiebbar auslenken oder verkippen, so dass der Stoppgegenanschlag dem Stoppanschlag gegenüberliegt. Mit anderen Worten ausgedrückt, heißt das, dass der Stoppgegenanschlag in einer ersten, unausgelenkten Position einen anderen radialen Abstand zu der Nebendrehachse oder zur Hauptdrehachse aufweist als der Stoppanschlag. In der zweiten, ausgelenkten Position des Stoppgegenanschlags weisen der Stoppgegenanschlag und der Stoppanschlag in etwa den gleichen Abstand zur Nebendrehachse auf, so dass sie sich gegenüberliegen, wodurch bewirkt wird, dass der Stoppgegenanschlag an dem Stoppanschlag anschlägt oder gegen den Stoppanschlag gedrückt wird, wenn das erste Element in die erste Drehrichtung gedreht wird. Der Vorteil an einem auslenkbaren oder verkippbaren Stoppgegenanschlag ist, dass der Stoppgegenanschlag mindestens einmal oder sogar mehrfach an dem Stoppanschlag vorbei gedreht werden kann und erst dann, wenn die Schaltstruktur mit der Schaltgegenstruktur in einen Kontakt kommt oder allgemein die Schaltstruktur den Stoppgegenanschlag aus der ersten, unausgelenkten Position in die zweite, ausgelenkte Position bewegt, an dem Stoppanschlag anschlagen kann.

Der Stoppgegenanschlag kann zum Beispiel an einem elastischen Arm, der sich zum Beispiel in Umfangsrichtung des Stoppelements erstreckt, an dem Stoppelement gebildet werden.

Beispielsweise kann die erste Eingriffsstruktur am Außenumfang des Stoppelements gebildet werden, wobei die zweite Eingriffsstruktur am Innenumfang des ersten Elements oder des zweiten Elements gebildet sein kann. Wenn die zweite Eingriffsstruktur am Innenumfang des ersten Elements gebildet ist, können die erste Gleitfläche am Innenumfang des Stoppelements und die zweite Gleitfläche am Außenumfang des zweiten Elements gebildet sein. Wenn die zweite Eingriffsstruktur am Innenumfang des zweiten Elements gebildet ist, können die erste Gleitfläche am Innenumfang des Stoppelements und die zweite Gleitfläche am Außenumfang des ersten Elements gebildet sein.

Alternativ kann die erste Eingriffsstruktur am Innenumfang des Stoppelements angeordnet sein, wobei die zweite Eingriffsstruktur am Außenumfang des ersten Elements oder des zweiten Elements gebildet sein kann. Wenn die zweite Eingriffsstruktur am Außenumfang des ersten Elements gebildet wird, kann die erste Gleitfläche am Außenumfang des Stoppelements und die zweite Gleitfläche am Innenumfang des zweiten Elementes gebildet werden. Wenn die zweite Eingriffsstruktur am Außenumfang des zweiten Elements gebildet ist, kann die erste Gleitfläche am Außenumfang des Stoppelements und die zweite Gleitfläche am Innenumfang des ersten Elements gebildet werden.

In einem bevorzugten Beispiel kann die zweite Eingriffsstruktur über den Innenumfang des ersten Elements angeordnet sein, wobei die erste Eingriffsstruktur über den Außenumfang des Stoppelements angeordnet sein kann. Die erste Eingriffsstruktur kann einen ersten Abschnitt und einen zweiten Abschnitt aufweisen, die entlang der Nebendrehachse zueinander versetzt und z.B. beabstandet sind. Der Stoppgegenanschlag kann zwischen dem ersten Abschnitt und dem zweiten Abschnitt angeordnet sein, insbesondere an dem Arm, wie zum Beispiel an einer elastischen Zunge. Das zweite Element kann zum Beispiel den Stoppanschlag bilden und/oder das Stoppelement kann das zweite Element umgeben.

In einem weiteren bevorzugten Beispiel kann die zweite Eingriffsstruktur über den Innenumfang des ersten Elements angeordnet sein, wobei die erste Eingriffsstruktur über den Außenumfang des Stoppelements angeordnet sein kann, wobei das hülsenförmige Stoppelement entlang der Nebendrehachse einen teilweise oder bevorzugt durchgängigen Schlitz, d. h. vom proximalen Ende bis zum distalen Ende des hülsenförmigen Stoppelements durchgängigen Schlitz, aufweist. Eine der den Schlitz des Stoppelements einfassenden Wände, d. h. die Schlitzwände, kann den Stoppgegenanschlag, insbesondere die Stoppgegenanschlagsfläche bilden. Bevorzugt ist, dass das zweite Element den Stoppanschlag bildet und/oder dass das Stoppelement das zweite Element umgibt.

In einem weiteren bevorzugten Beispiel kann die zweite Eingriffsstruktur über den Außenumfang des zweiten Elements angeordnet sein, wobei die erste Eingriffsstruktur über den Innenumfang des Stoppelements angeordnet ist. Ähnlich wie beim vorhergehenden Beispiel kann das hülsenförmige Stoppelement entlang der Nebendrehachse einen vollständigen oder bevorzugt teilweise durchgängigen Schlitz aufweisen, wobei eine der den Schlitz einfassenden Wände des Stoppelements den Stoppgegenanschlag bildet. Bevorzugt ist, dass das erste Element den Stoppanschlag bildet oder dass das Stoppelement das zweite Element umgibt.

Insbesondere kann das Stoppelement eine an seinem Innenumfang angeordnete, von der ersten Eingriffsstruktur abgesetzte Schaltgegenstruktur, insbesondere in der Gestalt einer Abragung, aufweisen. Das zweite Element kann ein von der zweiten Eingriffsstruktur abgesetzte Schaltstruktur, insbesondere in der Gestalt einer zweiten Abragung, aufweisen. Die Schaltstruktur, insbesondere die zweite Abragung, kann die Schaltgegenstruktur, insbesondere die erste Abragung, und den Stoppgegenanschlag während der Drehung des ersten Elements quer zur Umfangsrichtung elastisch auslenken, nämlich dann, wenn die Schaltstruktur die Schaltgegenstruktur kontaktiert, so dass der Stoppgegenanschlag dem von dem ersten Element gebildeten Stoppanschlag gegenüberliegt.

In einem weiteren Beispiel kann die zweite Eingriffsstruktur über den Innenumfang des zweiten Elements angeordnet sein, wobei die erste Eingriffsstruktur über den Außenumfang des Stoppelements angeordnet sein kann. Der Stoppgegenanschlag kann entlang der Nebendrehachse versetzt zu der ersten Eingriffsstruktur angeordnet sein, insbesondere an dem elastischen Arm, wie zum Beispiel an einer elastischen Zunge. Bevorzugt ist, dass das zweite Element den Stoppanschlag bildet und/oder dass das Stoppelement das erste Element umgibt.

In noch einem weiteren Beispiel kann die zweite Eingriffsstruktur über den Außenumfang des zweiten Elements angeordnet sein, wobei die erste Eingriffsstruktur über den Innenumfang des Stoppelements angeordnet sein kann, wobei die zweite Eingriffsstruktur eine Vielzahl über den Umfang angeordnete Führungsbahnen aufweist, die jeweils ein stirnseitiges Ende aufweisen. Das Ende mindestens einer der Führungsbahnen kann entlang der Hauptdrehachse oder der Nebendrehachse versetzt zu dem Enden der anderen Führungsbahnen angeordnet sein. Das Ende der mindestens einen Führungsbahn kann somit in Bezug auf die Enden der anderen Führungsbahnen bezogen auf die Haupt- oder Nebendrehachse auf unterschiedlichen Positionen angeordnet sein. Die Führungsbahnen können sich parallel zu oder helixförmig um die Haupt- oder Nebendrehachse erstrecken. Die erste Eingriffsstruktur oder das Stoppelement kann ein Eingriffsglied aufweisen, wobei das Stoppelement entlang der Hauptdrehachse oder der Nebendrehachse relativ zu dem zweiten Element bewegbar ist, wenn das Eingriffsglied in der mindestens einen Führungsbahn ist, deren Ende versetzt zu den anderen bzw. übrigen Enden der anderen bzw. übrigen Führungsbahnen angeordnet ist. Wenn das Eingriffsglied in dieser mindestens einen Führungsbahn ist, kann das Stoppelement entlang der Neben- oder Hauptdrehachse verschoben werden, wodurch auch der Stoppgegenanschlag verschoben wird. Durch das Verschieben kann der Stoppgegenanschlag in eine dem Stoppanschlag gegenüberliegende Position verschoben werden oder verschiebbar sein. Der Stoppanschlag kann zum Beispiel an dem ersten Element bzw. an einem Innenumfang des ersten Elements gebildet sein. Das Eingriffsglied kann zum Beispiel dem einen Punkt und die Führungsbahn dem anderen Punkt entsprechen, wobei die Punkte ihre Führungsbahnen, insbesondere kreisförmigen Führungsbahnen, mehrfach durchlaufen können, bis sie in einen gegenseitigen Kontakt kommen, der bewirkt, dass das Stoppelement dann verschoben werden kann. Der Stoppgegenanschlag kann somit an dem Stoppanschlag zum Beispiel mehrfach vorbeibewegt werden, bis das Eingriffsglied in die mindestens eine Führungsbahn eingreift, wodurch es entlang der Haupt- oder Nebendrehachse verschoben wird, wodurch der Stoppanschlag und der Stoppgegenanschlag in Umfangsrichtung gegenüberliegen.

Insbesondere kann zwischen dem ersten Element und dem Stoppelement eine vorgespannte Feder, insbesondere eine Druckfeder angeordnet sein, welche das Stoppelement zu dem versetzten Ende hin verschiebt, wenn das Eingriffsglied in der mindestens einen Führungsbahn ist, deren Ende versetzt ist.

Alternativ oder zusätzlich kann die zweite Eingriffsstruktur, insbesondere die Führungsbahn, eine Schrägverzahnung sein, und das erste Element und das Stoppelement in einem Reibeingriff stehen. Vorzugsweise sind die erste und zweite Gleitfläche so aneinander angepasst, dass sie eine etwas erhöhte Reibung im Vergleich zu glatten Flächen erzeugen. Dies kann zum Beispiel dadurch bewirkt werden, dass die erste und zweite Gleitfläche derart strukturiert sind, dass sie ineinandergreifen aber dennoch um die Nebendrehachse aneinander abgleiten können. Das erste Element kann das Stoppelement aufgrund des Reibeingriffs mitdrehen, wenn das Eingriffsglied in der mindestens einen Führungsbahn ist, deren Ende versetzt angeordnet ist, vorausgesetzt das erste Element wird in die erste Drehrichtung gedreht. Dadurch folgt das Eingriffsglied der helixförmigen Führungsbahn, wodurch das Stoppelement an der mindestens einen helixförmigen Führungsbahn entlang bewegt wird, insbesondere mit einer kombinierten Dreh- und Axialbewegung. Hierdurch wird insbesondere bewirkt, dass das Stoppelement zu dem versetzen Ende hin bewegt wird. Dadurch kann der Stoppgegenanschlag in eine Position verschoben werden, in der er dem Stoppanschlag in Umfangsrichtung gegenüberliegt.

Wenn der Stoppgegenanschlag dem Stoppanschlag gegenüberliegt, wird allgemein bewirkt, dass der Stoppgegenanschlag in Umfangsrichtung gegen den Stoppanschlag gedrückt wird, wenn versucht wird, das erste Element in die erste Drehrichtung zu drehen. Hierbei wird eine Drehung des ersten Elements in die erste Drehrichtung blockiert.

In einem weiteren Beispiel, kann die erste Eingriffsstruktur eine Stirnverzahnung und vorzugsweise zusätzlich eine Innenverzahnung umfassen oder sein. Die zweite Eingriffsstruktur kann eine Stirnverzahnung sein, wobei bevorzugt ist, dass das hülsenförmige Stoppelement entlang der Nebendrehachse einen teilweise oder bevorzugt durchgängigen Schlitz aufweist, wobei eine der den Schlitz einfassenden Wände des Stoppelements den Stoppgegenanschlag bildet. In dieser Ausführungsform ist besonders bevorzugt, dass die Nebendrehachse winkelversetzt zu der Hauptdrehachse steht.

In einem zweiten Aspekt weist das hülsenförmige Stoppelement, über welches das erste Element und das zweite Element gekoppelt sind, eine Innenverzahnung auf, wobei das Stoppelement einen Stoppgegenanschlag bildet. Das erste Element und das zweite Element sind ferner über eine flexible Hülse gekoppelt, welche eine Außenverzahnung aufweist, welche in die Innenverzahnung des hülsenförmigen Stoppelements eingreift. Das erste Element bildet vorzugsweise zwei Gleitflächen, welche die flexible Hülse oval aufspannen. Vorzugsweise liegen die zwei Gleitflächen des ersten Elements an dem Innenumfang der flexiblen Hülse an. Wenn das erste Element relativ zu dem zweiten Element in die erste Drehrichtung oder in die zweite Drehrichtung gedreht wird, gleiten die zwei Gleitflächen am Innenumfang der flexiblen Hülse ab. Dadurch wird die flexible Hülse verformt, wobei sie insbesondere oval aufgespannt bleibt. In den Bereichen, in denen die zwei Gleitflächen an der flexiblen Hülse anliegen, wird die Außenverzahnung der flexiblen Hülse in die Innenverzahnung gedrückt. In den übrigen Bereichen ist die Außenverzahnung nicht im Eingriff mit der Innenverzahnung. Die Zähnezahl der Außenverzahnung ist insbesondere geringer, wie zum Beispiel um ein, zwei oder drei Zähne geringer als die Zähnezahl der Innenverzahnung.

Hierdurch wird die Drehung des ersten Elements relativ zu dem zweiten Element unter Drehrichtungsumkehr an das hülsenförmige Stoppelement übersetzt. Das hülsenförmige Stoppelement dreht sich somit in die zweite Drehrichtung, wenn das erste Element in die erste Drehrichtung gedreht wird, wobei sich das Stoppelement in die erste Drehrichtung dreht, wenn das erste Element in die zweite Drehrichtung gedreht wird.

Insbesondere ist an dem zweiten Element der Stoppanschlag gebildet, wobei der Stoppgegenanschlag während der Drehung des ersten Elements in die erste Drehrichtung eine Bewegung zu der Stoppposition hin ausführt, insbesondere in die zweite Drehrichtung, wobei in der Stoppposition der Stoppgegenanschlag an dem Stoppanschlag anschlägt und die Drehung des ersten Elements relativ zu dem zweiten Element in die erste Drehrichtung verhindert. Der vollständig halber sei erwähnt, dass der Stoppgegenanschlag während der Drehung des ersten Elements in die zweite Drehrichtung eine Bewegung von der Stoppposition weg ausführt.

Das hierdurch in dem Dosiermechanismus gebildete Getriebe ist in Anlehnung an einem Gleitkeilgetriebe ausgestaltet, welches auch unter der Bezeichnung "harmonic drive®" bekannt ist.

Sowohl für den ersten als auch zweiten Aspekt ist bevorzugt, dass das Dosiseinstellelement drehbar ist. Es kann relativ zu dem Gehäuse axialfest oder längsbewegbar sein. Zum Beispiel kann das Dosiseinstellelement bei der Drehbewegung eine Längsbewegung, wie zum Beispiel eine Schraubbewegung ausführen.

Das Dosiseinstellelement kann hülsenförmig sein und sich zum Beispiel im Bereich des proximalen Endes der Injektionsvorrichtung befinden. Alternativ oder zusätzlich kann das Dosiseinstellelement innerhalb des Gehäuses der Vorrichtung angeordnet sein, wobei Gehäuse und Dosiseinstellelement hülsenförmig sein können. Denkbar ist, dass der Verwender nicht unmittelbar Zugriff auf das Dosiseinstellelement hat, sondern über zusätzliche Teile. Grundsätzlich kann das Dosiseinstellelement ein mehrteilig aus sich zum Beispiel rotatorisch oder translatorisch bewegenden Teilen gebildet sein, wobei auch ein einteiliges Dosiseinstellelement denkbar ist. Das sich innerhalb des Gehäuse befindliche Dosiseinstellelement kann zum Beispiel eine Dosisanzeigehülse sein, die zum Beispiel durch eine Öffnung oder ein Fenster von außen ablesbar ist. Allgemein kann das Dosiseinstellelement mit einer Dosisanzeigehülse verbunden oder gekoppelt sein.

Das erste Element und/oder zweite Element sind bevorzugt hülsenförmig oder/und können um eine gemeinsame Drehachse, insbesondere eine Hauptdrehachse, drehen, wobei diese Drehachse beispielsweise in Längsrichtung der Injektionsvorrichtung oder Richtung der Nadel weist. Das erste Element und das zweite Element können konzentrisch zueinander angeordnet sein.

Das erste Element kann in beispielhaften Ausführungsformen mit einer Feder, insbesondere einer Torsionsfeder oder Drehfeder verbunden sein, die die zur Dosisausschüttung notwendige Energie speichert und bei Bedarf abgibt. Bevorzugt kann durch die Dosiseinstellbewegung des Dosiseinstellelements oder des ersten Elements die Feder gespannt werden. Grundsätzlich kann die Feder wendelförmig oder vorzugsweise spiralförmig sein. Die Feder kann aus einem Draht oder vorzugsweise aus einem bandförmigen Material, insbesondere Federstahl gewickelt sein. Solche Federn werden auch als Uhrenfedern bezeichnet. Wenigstens eines aus Dosisanzeigehülse, erstem Element und Dosiseinstellelement können bei der Dosiseinstellung drehfest mit einem Ende der Feder gekoppelt sein, wobei das andere Ende der Feder vorteilhaft mit dem Gehäuse verbunden sein kann. Für die Dosisausschüttung können das erste Element und die Dosisanzeigehülse drehfest mit einem Ende der Feder gekoppelt sein oder werden, wobei das Dosiseinstellelement beispielsweise entkoppelt ist.

Die Dosisanzeigehülse kann zum Beispiel ein Gewinde, insbesondere ein Innen- oder Außengewinde aufweisen, das in das Gehäuse oder in ein gehäusefestes Element eingreift, um für die Dosisanzeige eine Schraubbewegung ausführen zu können. Die Dosisanzeigehülse kann eine an ihrem Außenumfang angeordnete, der Gewindesteigung entsprechend wendelförmige Skala aufweisen. Die Dosisanzeigehülse kann Anschläge, insbesondere in Axialrichtung oder vorzugsweise in Umfangsrichtung wirkende Anschläge aufweisen, die in entsprechende Gegenanschläge, wie zum Beispiel des Gehäuses anschlagen können. Die Dosisanzeigehülse kann sich zwischen diesen Anschlägen hin und her bewegen, so dass mit ihr Dosen von 0 bis zur gewünschten Maximaldosis, wie zum Beispiel 60 oder 80 internationale Einheiten einstellbar sind, insbesondere sofern sich das Stoppelement nicht in der Stoppposition befindet. Somit kann eine maximal einstellbare Dosis einerseits mit dem Anschlag der Dosisanzeigehülse oder andererseits mit dem Stoppelement begrenzt werden, je nach dem welches der beiden Elemente zuerst eine Dosiserhöhung, d. h. eine Drehung des Dosiseinstellelements oder des ersten Elements in die erste Drehrichtung verhindert.

Allgemein bevorzugt kann das zweite Element drehfest so mit einem Abtriebsglied, insbesondere einer Kolbenstange oder Gewindestange gekoppelt sein, wobei das Abtriebsglied insbesondere für eine Produktausschüttung in die Ausschüttrichtung schraubbar ist. Hierzu kann das Abtriebsglied beispielsweise ein Gewinde aufweisen, mit der es in das Gehäuse oder ein gehäusefestes Element eingreift, um die Schraubbewegung ausführen zu können. Beispielsweise kann das zweite Element hülsenförmig und verdrehfest und axialverschiebbar mit dem Abtriebsglied gekoppelt sein. Das Abtriebsglied kann eine Längsbewegung relativ zu dem zweiten Element ausführen. Das zweite Element kann mittelbar oder unmittelbar mit dem Abtriebsglied gekoppelt sein, insbesondere in einem unmittelbaren Eingriff mit dem Abtriebsglied sein. Beispielsweise kann das zweite Element über eine Hülse, insbesondere eine Kupplungshülse drehfest oder längsverschiebbar mit dem Abtriebsglied gekoppelt sein. Das zweite Element kann drehfest und längsverschiebbar mit der Hülse, die zwischen dem Abtriebsglied und dem zweiten Element angeordnet sein kann, gekoppelt sein, wie zum Beispiel über eine Längsnut. Dies hat den Vorteil, dass bei einem Produktbehälterwechsel das zweite Element relativ zum ersten Element keine Axialbewegung ausführen braucht oder ausführt, wobei das Abtriebsglied und ggf. die das Abtriebsglied umgebende Hülse axial relativ zu dem zweiten Element bewegbar sein können.

Die Erfindung wurde an Hand einer Vielzahl bevorzugter Ausführungen und Beispielen Beschrieben. Im Folgenden werden besonders bevorzugte Ausführungen anhand von Figuren beschreiben. Die dabei offenbarten Merkmale bilden den Gegenstand der unabhängigen Ansprüche einzeln und in jeglicher Merkmalskombination vorteilhaft weiter. Es zeigen:
- Figur 1: eine Explosionsdarstellung der wesentlichen Teile einer ersten Ausführungsform der Erfindung,
- Figur 2: verschiedene Ansichten einer Injektionsvorrichtung, welche den erfindungsgemäßen Dosiermechanismus der ersten Ausführungsform in einem Ausgangszustand aufweist,
- Figur 3: die Ansichten aus Figur 2, wobei ein Stoppgegenanschlag an einem Stoppanschlag anschlägt und die weitere Dosiserhöhung blockiert,
- Figur 4: eine perspektivische Schnittansicht des Schnitts H-H aus Figur 3,
- Figur 5: verschiedene Ansichten einer zweiten Ausführungsform,
- Figuren 6a - 7: verschiedene Ansichten einer dritten Ausführungsform,
- Figuren 8 - 9: verschiedene Ansichten einer vierten Ausführungsform,
- Figur 10: eine Explosionsdarstellung der wesentlichen Teile einer fünften Ausführungsform,
- Figur 11: verschiedene Ansichten der Ausführung aus Figur 10 in einer Ausgangsposition,
- Figur 12: die Ansichten aus Figur 11 in einer Stoppposition,
- Figuren 13a - 13b: verschiedene Ansichten der wesentlichen Teile einer sechsten Ausführungsform,
- Figur 14: die Ausführungsform aus den Figuren 13a und 13b in verschiedenen Ansichten in einer Anfangsposition,
- Figur 15: die Ansichten aus Figur 14 in einer Stoppposition,
- Figuren 16a - 16b: verschiedene Ansichten der wesentlichen Teile einer siebten Ausführungsform,
- Figur 17: verschiedene Ansichten der Ausführungsform aus den Figuren 16a und 16b in einer Anfangsposition,
- Figur 18: die Ansichten aus Figur 17 in einer Stoppposition,
- Figur 19: eine perspektivische Ansicht der wesentlichen Teile einer achten Ausführungsform,
- Figur 20: verschiedene Ansichten der Ausführungsform aus Figur 19 in einer Anfangsposition,
- Figur 21: die Ansichten aus Figur 20 in einer Stoppposition,
- Figur 22a: eine Explosionsdarstellung der wesentlichen Teile einer neunten Ausführungsform,
- Figur 22b: die Ansicht aus Figur 22a, wobei die Einzelteile im Schnitt dargestellt sind,
- Figur 23: verschiedene Ansichten der Ausführungsform aus den Figuren 22a und 22b,
- Figur 24: eine perspektivische Ansicht der wesentlichen Teile einer zehnten Ausführungsform,
- Figur 25a: verschiedene Ansichten der Ausführungsform aus Figur 24 in einer Anfangsposition und,
- Figur 25b: die Ansichten aus Figur 25a in einer Stoppposition.

Der erfindungsgemäße Dosiermechanismus lässt sich im Allgemeinen an jeder Stelle einer Injektionsvorrichtung realisieren, in der ein erstes Element 100 und ein zweites Element 200 vorhanden sind, wobei das erste Element 100 während einer Dosiseinstellung relativ zu dem anderen, zweiten Element 200 um eine Drehachse H in eine erste Drehrichtung gedreht wird und während einer Dosisausschüttung relativ zu dem zweiten Element 200 drehfest ist bzw. rotatorisch still steht. Diese ersten und zweiten Elemente 100, 200 können über ein hülsenförmiges Stoppelement 300, welches einen Stoppgegenanschlag 310 bildet, gekoppelt sein.

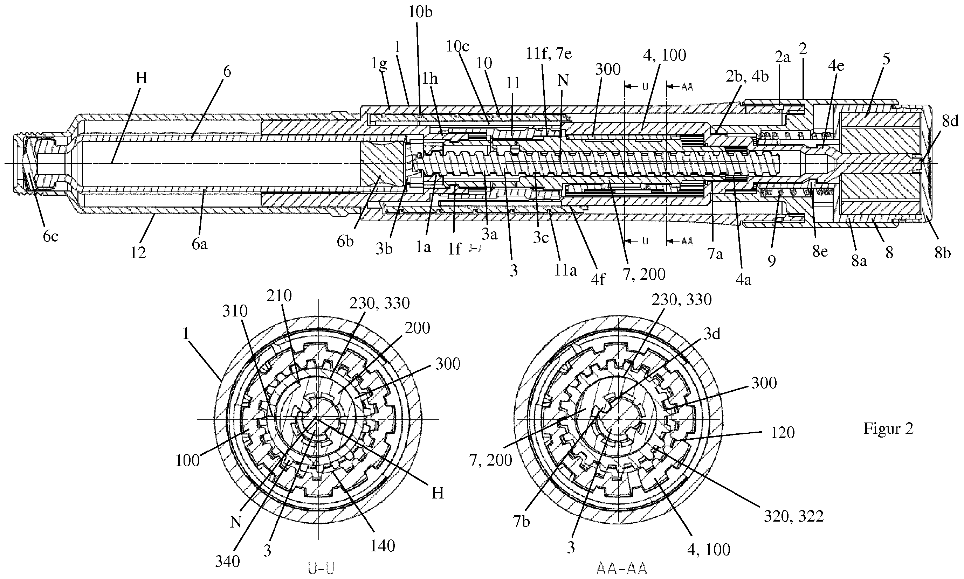

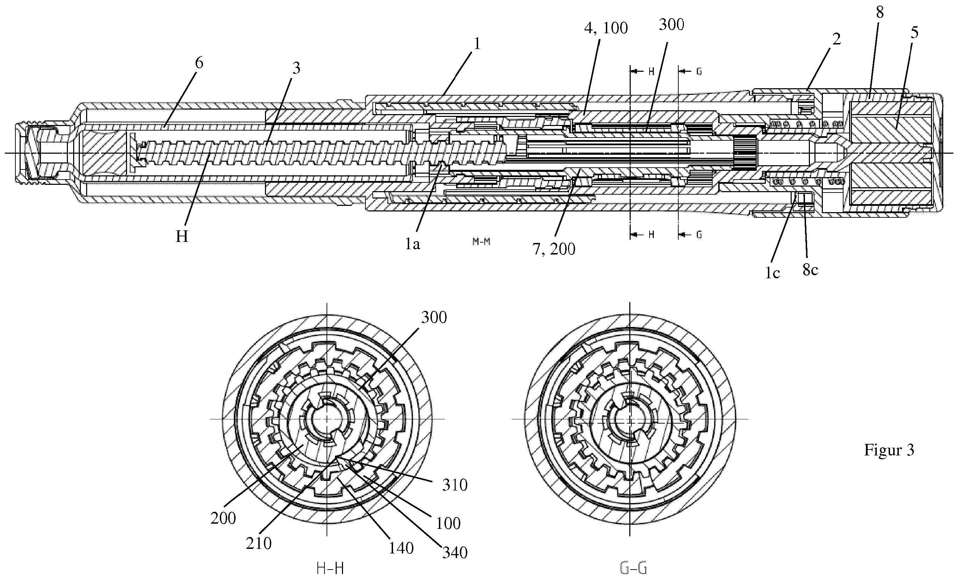

In diesem Sinne ist die in den Figuren 2 und 3 gezeigte Injektionsvorrichtung lediglich als ein vorteilhaftes Beispiel zu verstehen, da sich die Erfindung auch in einer Vielzahl anderer Injektionsvorrichtungen einsetzen lässt, solange das erste Element 100 relativ zu dem zweiten Element 200 während der Dosiseinstellung gedreht wird und während der Produktausschüttung in Bezug auf das zweite Element 200 rotatorisch still steht.

Mit Bezug auf die Figuren 2 und 3 wird der Dosiermechanismus als Antriebs- und Dosiervorrichtung bezeichnet und bildet eine Injektionsvorrichtung oder ist zumindest ein Teil einer solchen Injektionsvorrichtung. Die Antriebs- und Dosiervorrichtung weist ein hülsenförmiges Gehäuse 1 auf, welches eine Außenhülse 1g und eine damit verbundene und konzentrisch dazu angeordnete Innenhülse 1h aufweist. Die Innenhülse 1h und die Außenhülse 1g sind über einen ringförmigen Steg fest verbunden. Das Gehäuse 1, insbesondere die Innenhülse 1h weist einen Innengewinde 1a auf, welches in ein Außengewinde 3c der Gewindestange 3a eingreift, so dass die Gewindestange 3a und somit das Abtriebsglied 3, relativ zu dem Gehäuse 1 um und entlang der (Haupt-) Drehachse H in die distale Richtung geschraubt werden kann. Das Abtriebsglied 3 weist die Gewindestange 3a und einen tellerförmigen Flansch 3b auf, der frei drehbar am distalen Ende der Gewindestange 3a befestigt, insbesondere verschnappt ist. Die Gewindestange 3a weist mindestens eine Führungsnut 3d auf, welche das Außengewinde 3c überlagert und sich parallel zur Hauptdrehachse H erstreckt. Ein hülsenförmiges Rotationsglied 7, welches dem zweiten Element 200 im Sinne der Erfindung entspricht, weist an seinem Innenumfang mindestens ein stegförmiges Eingriffsglied 7b auf, welches in die Führungsnut 3d eingreift, wodurch das Rotationsglied 7 und das Abtriebsglied 3 relativ zueinander verdrehfest und axial verschiebbar sind. Das Rotationsglied 7 weist an seinem Außenumfang eine ringförmige Nut 7d auf, in welche eine am Innenumfang des Gehäuses 1, insbesondere der Innenhülse 1h gebildete Abragung 1f eingreift, wodurch das Rotationsglied 7 relativ zu dem Gehäuse 1 verdrehbar und axialfest ist. Eine Drehung des Rotationsglieds 7 bewirkt eine Drehung des Abtriebsglieds 3, wodurch das Abtriebsglied 3 aufgrund des Gewindeeingriffs mit dem Gehäuse 1 entlang der Hauptdrehachse H bewegbar ist. Beispielsweise wird das Abtriebsglied 3 in die distale Richtung bewegt, wenn das Rotationsglied 7 relativ zu dem Gehäuse 1 in eine zweite Drehrichtung um die Hauptdrehachse H gedreht wird.

Das Rotationsglied 7 weist eine zweite Kupplungsstruktur 7a in Gestalt einer Außenverzahnung auf.

Das Gehäuse 1, insbesondere die Innenhülse 1h weist an ihrem Außenumfang eine Eingriffsstruktur auf, wobei diese Eingriffsstruktur und eine am Innenumfang des Verschiebeglieds 11 gebildete Nut oder rippenförmige Längsführung 11 e so ineinandergreifen, dass das Verschiebeglied 11 relativ zu dem Gehäuse 1 um die Hauptdrehachse H verdrehgesichert und entlang der Hauptdrehachse H verschiebbar ist. Das Verschiebglied 11 weist an seinem Innenumfang eine Innenverzahnung auf, in welche ein Rastmittel 7c, welches federnd an dem Rotationsglied 7 gebildet ist, eingreift. Das Rastmittel 7c weist einen Rastarm auf, an dessen Außenseite eine Eingriffsnocke angeordnet ist, welche in die Innenverzahnung eingreift. Bei der Drehung des Rotationsglieds 7 relativ zu dem Verschiebeglied 11 gleitet das Rastmittel 7c über die Innenverzahnung, wodurch zum Beispiel mittels eines akustischen und/oder taktilen Signals die Produktausschüttung signalisiert werden kann.

Der Eingriff des Rastmittels 7c in die Innenverzahnung kann in einer Variante derart sein, dass das Rotationsglied 7 nur nach Überwindung eines gewissen Grenzdrehmoments relativ zu dem Gehäuse 1 oder dem Verschiebeglied 11 gedreht werden kann. Hierdurch verhindert der Eingriff des Rastmittels 7c in die Innenverzahnung 11f, dass sich das Abtriebsglied 3 unbeabsichtigt relativ zu dem Gehäuse 1 verdreht, wie zum Beispiel durch Vibrationen beim Transport der Antriebs- und Dosiervorrichtung. Durch Abstimmung des Rastmittels 7c und der Innenverzahnung ist das Grenzdrehmoment so ausgelegt, dass es ohne weiteres von einem Drehmoment, welches durch eine rotatorisch vorgespannte Antriebsfeder 5 während der Produktausschüttung bereitgestellt wird, überwunden werden kann.

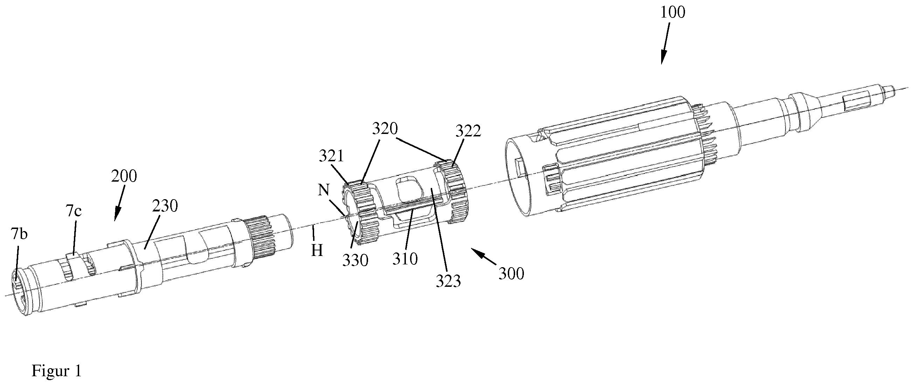

In den Figuren 2 und 3 wird jedoch eine andere, bevorzugte Variante gezeigt, in der das Rastmittel 7c (Figur 1) die Innenverzahnung lediglich zur Erzeugung eines akustischen und/oder taktilen Signals während der Produktausschüttung dienen. Das Verschiebeglied 11 bildet eine siebte Kupplungsstruktur 11f. Das Rotationsglied 7 bildet eine achte Kupplungsstruktur 7e in der Gestalt einer Verzahnung, insbesondere Außenverzahnung, die separat von der zweiten Kupplungsstruktur 7a ist. Die siebte Kupplungsstruktur 11f und die achte Kupplungsstruktur 7e bilden die vierte Kupplung 7e, 11f.

Die vierte Kupplung 7e, 11f ist eingekuppelt, wenn das Betätigungsglied 8 unbetätigt ist, und ausgekuppelt, wenn das Betätigungsglied 8 betätigt, d. h. gedrückt ist. Wenn die vierte Kupplung 7e, 11f eingekuppelt ist, greifen die siebte Kupplungsstruktur 11f und die achte Kupplungsstruktur 7e verdrehfest ineinander.

Das Verschiebeglied 11 weist an seinem proximalen Ende eine Ringnut auf, in welche eine Abragung am Innenumfang eines Antriebsglieds 4, welches dem ersten Element 100 im Sinne der Erfindung entspricht, eingreift, wodurch das Antriebsglied 4 relativ zu dem Verschiebeglied 11 verdrehbar und axialfest ist. Eine Verschiebung des Antriebsglieds 4 entlang der Hauptdrehachse H bewirkt somit auch eine Verschiebung des Verschiebeglieds 11 entlang der Hauptdrehachse H. Das Antriebsglied 4 weist eine erste Kupplungsstruktur 4a in Gestalt einer Innenverzahnung auf, welche mit der zweiten Kupplungsstruktur 7a eine erste Kupplung 4a, 7a bildet. Das Antriebsglied 4 ist aus einer ausgekuppelten Position, in welcher die erste Kupplungsstruktur 4a und die zweite Kupplungsstruktur 7a nicht ineinandergreifen, in eine eingekuppelte Position entlang der Hauptdrehachse H verschiebbar, in welcher die erste Kupplungsstruktur 4a und die zweite Kupplungsstruktur 7a formschlüssig ineinandergreifen. Das Antriebsglied 4 ist relativ zu dem Rotationsglied 7 um die Hauptdrehachse H drehbar, wenn die erste Kupplung 4a, 7a ausgekuppelt ist, und relativ zu dem Rotationsglied 7 um die Hauptdrehachse verdrehfest, wenn die erste Kupplung 4a, 7a eingekuppelt ist.

Das Antriebsglied 4 weist eine Raststruktur auf, welche federnd an dem Antriebsglied 4 gebildet ist. Die Raststruktur weist mindestens einen Zahn auf, welcher in einem Eingriff mit einer Verzahnung, insbesondere der zweiten Kupplungsstruktur 7a des Rotationsglieds 7 ist, wenn die erste Kupplung 4a, 7a ausgekuppelt ist. Die Raststruktur 4d rastet über die Verzahnung, insbesondere die zweite Kupplungsstruktur 7a des Rotationsglieds 7, wenn das Antriebsglied 4 relativ zu dem Rotationsglied 7 während der Einstellung einer auszuschüttenden Produktdosis in eine erste Drehrichtung oder/und eine zweite Drehrichtung verdreht wird. Hierdurch werden einerseits Klickgeräusche erzeugt, welche dem Verwender das Einstellen der Dosis taktil und/oder akustisch signalisieren und andererseits diskrete Winkelpositionen für das Antriebsglied 4 in Bezug auf das Rotationsglied 7 vorgegeben.

Das Antriebsglied 4 weist eine vierte Kupplungsstruktur 4b auf, die als Außenverzahnung ausgestaltet ist. Die vierte Kupplungsstruktur 4b bildet mit einer dritten Kupplungsstruktur 2b, die als Innenverzahnung ausgestaltet ist, eine zweite Kupplung 2b, 4b. An dem Gehäuse 1 ist ein hülsenförmiges Dosiseinstellglied 2 befestigt, wobei das Dosiseinstellglied 2 relativ zu dem Gehäuse 1 drehbar und axialfest ist. Das Dosiseinstellglied 2 weist eine Außenhülse und eine Innenhülse auf, die über einen Steg fest miteinander verbunden sind. Das Dosiseinstellglied 2, insbesondere die Außenhülse weist an ihrem Innenumfang eine Abragung 2a auf, welche in eine Ringnut des Gehäuses 1, insbesondere der Außenhülse 1g eingreift, so dass das Dosiseinstellglied 2 relativ zu dem Gehäuse 1 drehbar und axialfest ist.

Das Dosiseinstellglied 2, insbesondere deren Innenhülse, bildet die dritte Kupplungsstruktur 2b. Die dritte Kupplungsstruktur 2b greift formschlüssig in die vierte Kupplungsstruktur 4b, wenn die zweite Kupplung 2b, 4b eingekuppelt ist, wodurch das Dosiseinstellglied 2 um die Hauptdrehachse H drehfest mit dem Antriebsglied 4 verbunden ist. Das Antriebsglied 4 macht somit Drehbewegungen des Dosiseinstellglieds 2 mit. Wenn die zweite Kupplung 2b, 4b ausgekuppelt ist, greifen die dritte Kupplungsstruktur 2b und die vierte Kupplungsstruktur 4b nicht ineinander, so dass das Dosiseinstellglied 2 und das Antriebsglied 4 relativ zueinander verdrehbar sind.

Für die Einstellung einer auszuschüttenden Produktdosis wird das Dosiseinstellglied 2 relativ zu dem Gehäuse 1 zur Erhöhung der Dosis in eine erste Drehrichtung und zur Verringerung oder Korrektur der Dosis in eine zweite Drehrichtung gedreht. Während der Dosiseinstellung ist die zweite Kupplung 2b, 4b eingekuppelt, wodurch das Antriebsglied 4 die Drehbewegungen des Dosiseinstellglieds 2 mitmacht.

Das Dosiseinstellglied 2 ist am proximalen Ende des Gehäuses 1 angeordnet und ist vom Verwender der Vorrichtung greifbar und relativ zu dem Gehäuse 1 verdrehbar.

Das proximale Ende der Antriebs- und Dosiervorrichtung wird von einem Betätigungsglied 8, welches als Betätigungsknopf ausgestaltet ist, gebildet. Das Betätigungsglied 8 kann aus einer unbetätigten Position (siehe zum Beispiel Figuren 2 und 3) in eine betätigte Position (nicht gezeigt) gegen eine Rücksetzfeder 9 verschoben werden, wobei hierdurch die Rücksetzfeder 9 gespannt wird. Die Rücksetzfeder 9 ist eine Wendel- oder Schraubenfeder, welche als Druckfeder wirkt und sich mit ihrem distalen Ende an dem Dosiseinstellglied 2 und mit seinem proximalen Ende an dem Betätigungsglied 8 abstützt. Beim Betätigen wird das Betätigungsglied 8 zum Beispiel mit dem Finger des Daumens der Hand, welche das Gehäuse 1 umgreift, gedrückt, wodurch die Feder 9 gespannt wird. Durch Loslassen das Betätigungsglied 8 kann die vorgespannte Feder 9 das Betätigungsglied 8 aus der betätigten Position in die unbetätigte Position zurückschieben.

Das Betätigungsglied 8 ist relativ zu dem Dosiseinstellglied 2 entlang der Hauptdrehachse H hin und her schiebbar, nämlich zwischen der betätigten Position und der unbetätigten Position. Das Betätigungsglied 8 ist mehrteilig ausgestaltet und weist ein Verbindungsglied 8a auf, welches einen hülsenförmigen Abschnitt aufweist, der am distalen Ende durch einen nach innen ragenden Kragen verengt oder verschlossen ist. Das proximale Ende des Verbindungsglieds 8a ist mittels einer Abdeckkappe 8b verschlossen, welche ebenfalls zu dem Betätigungsglied 8a gehört und eine Auflagefläche für den Daumen zum Betätigen des Betätigungsglieds 8 bildet.

Das Betätigungsglied 8, insbesondere der hülsenförmige Abschnitt des Verbindungsglieds 8a umgibt die Antriebsfeder 5 umfangsseitig. Die Antriebsfeder 5 ist eine aus einem bandförmigen Material spiralförmig gewickelte Feder, die auch als Uhrenfeder bezeichnet werden kann. Ein erster Abschnitt, insbesondere ein erstes Ende der Antriebsfeder 5 ist an dem Betätigungsglied 8, insbesondere an dessen zylindrischem Abschnitt befestigt. Ein zweiter Abschnitt, insbesondere ein zweites Ende der Antriebsfeder 5 ist an dem Antriebsglied 4, insbesondere zwischen dessen proximalem Ende 4c und einem Kragen 4e befestigt. Zwischen dem ersten Abschnitt und dem zweiten Abschnitt weist die Antriebsfeder 5 einen dritten Abschnitt auf, der bei Änderung der Federspannung elastisch verformt wird. Eine Drehung des Antriebsglieds 4 relativ zu dem Betätigungsglied 8 bewirkt eine Änderung der Federspannung, insbesondere eine Abnahme der Federspannung unter Abgabe der von der Feder 5 gespeicherten potentiellen Energie an das Antriebsglied 4 in Form von kinetischer, d. h. Rotationsenergie. Das Antriebsglied 4 weist einen insbesondere stab- oder stiftförmigen Abschnitt auf, der sich durch den Kragen am distalen Ende des Betätigungsglieds 8 in das Innere des Betätigungsglieds 8 und durch die Antriebsfeder 5 erstreckt. Das proximale Ende verjüngt sich zum proximalen Ende der Antriebs- und Dosiervorrichtung hin, zum Beispiel kugelförmig, kegelig oder kegelstumpfförmig. Das Betätigungsglied 8, insbesondere die Abdeckkappe 8e bildet eine Kontaktfläche 8d für das proximale Ende des Antriebsglieds 4.

Das Betätigungsglied 8, insbesondere das Verbindungsglied 8a weist eine sechste Kupplungsstruktur 8c in der Gestalt einer Außenverzahnung auf, die zum Beispiel an einer in distale Richtung abragenden Abragung gebildet ist. Die Abragung greift durch den Kragen, der die Innenhülse mit der Außenhülse des Dosiseinstellglieds 2 verbindet. Das Gehäuse 1, insbesondere die Außenhülse 1g weist eine fünfte Kupplungsstruktur 1c auf, die als Innenverzahnung gebildet ist und mit der sechsten Kupplungsstruktur 8c eine dritte Kupplung 1c, 8c bildet. Durch Betätigen des Betätigungsglieds 8 lässt sich die dritte Kupplung 1c, 8c einkuppeln und durch Loslassen des Betätigungsglieds 8a auskuppeln. Bei eingekuppelter dritter Kupplung 1c, 8c greift die sechste Kupplungsstruktur 8c formschlüssig in die fünfte Kupplungsstruktur 1c, wodurch das Betätigungsglied 8 und insbesondere auch das mit dem Betätigungsglied 8 drehfest verbundene Dosiseinstellglied 2, drehfest in Bezug auf das Gehäuse 1 ist. Das Betätigungsglied 8 und das Dosiseinstellglied 2 sind relativ zu dem Gehäuse 1 drehbar, wenn die dritte Kupplung 1c, 8c ausgekuppelt ist, wobei die sechste Kupplungsstruktur 8c und die fünfte Kupplungsstruktur 1c dann nicht ineinandergreifen.

An dem distalen Ende des Gehäuses 1 ist ein hülsenförmiger Produktbehälterhalter, insbesondere unlösbar befestigt, in dem ein Produktbehälter 6 aufgenommen ist. Der Produktbehälter 6 ist in dem gezeigten Beispiel eine Karpule. Der Produktbehälter 6 weist einen Behälterkörper 6a auf, der ein flüssiges, zu verabreichendes Produkt umgibt, wobei in dem Behälterkörper 6a proximal des Produkts ein verschiebbarer Kolben 6b angeordnet ist, der dichtend an der Innenwand des Behälterkörpers 6 anliegt. An dem distalen Ende des Behälterkörpers 6a ist ein Septum 6c gebildet, welches mittels einer Nadel, die an einem Gewinde 12c des Produktbehälterhalters 12 anbringbar ist, durchstochen werden kann. Zur Verschiebung des Kolbens 6b in Richtung Septum 6c wird das in dem Produktbehälter 6 enthaltende Produkt über die Nadel ausgeschüttet.

Der Produktbehälterhalter 12 weist ein Fenster auf, durch welches die in dem Produktbehälter 6 enthaltene Produktmenge optisch überwacht werden kann. Der Produktbehälterhalter 12 weist eine Ausnehmung auf, in die eine erste Eingriffsstruktur 1b am Innenumfang des Gehäuses 1, insbesondere der Außenhülse 1 eingeschnappt ist, wenn der Produktbehälterhalter 12 an dem Gehäuse 1 befestigt ist. Der Produktbehälterhalter 12 weist einen an seinem Au-βenumfang ringförmig umlaufenden Kragen auf, der an dem distalen Ende des Gehäuses 1, insbesondere der Außenhülse 1g anliegt, wenn der Produktbehälterhalter 12 an dem Gehäuse 1 befestigt ist. Der Produktbehälterhalter 12 weist an seinem Außenumfang eine Nocke auf, mit der eine hülsenförmige Kappe, welche über den Produktbehälterhalter 12 schiebbar ist, lösbar verschnappbar ist. Die Kappe kann somit wieder abgenommen werden und dient lediglich dem Schutz einer optional angebrachten Nadel und/oder des Medikaments vor Lichteinwirkung. Zur Verschnappung mit der Nocke weist die Kappe eine ringförmig am Innenumfang angebrachte Ausnehmung, insbesondere Nut auf.

Die Antriebs- und Dosiervorrichtung weist ein hülsenförmiges Stoppelement 300, welches auch als Dosisbegrenzer 13 bezeichnet werden kann, auf, welches das erste Element 100 bzw. Antriebsglied 4 mit dem zweiten Element 200 bzw. Rotationsglied 7 koppelt. An dem zweiten Element 200 ist ein Stoppanschlag 210 gebildet, von dem ein Stoppgegenanschlag 310 des Stoppelements 300 proportional zu der maximal aus dem Produktbehälter 6 oder proportional zu einer vorgegebenen aus dem Produktbehälter 6 ausschüttbaren Produktmenge beabstandet ist. Da bei der Dosiseinstellung das Antriebsglied 4 relativ zu dem Rotationsglied 7 verdreht und bei einer Dosisausschüttung nicht verdreht wird, kann durch das Stoppelement 300 ein Zählwerk gebildet werden, welches die bereits ausgeschütteten Einzeldosen und die aktuell eingestellte Dosis addiert und sich dementsprechend immer näher an den Stoppanschlag 210 annähert. Eine Dosiserhöhung bewirkt, dass der Stoppgegenanschlag 310 zu dem Stoppanschlag 210 hinbewegt wird. Eine Dosisverringerung bewirkt, dass der Stoppgegenanschlag 310 von Stoppanschlag 210 wegbewegt wird. Ist die in dem Produktbehälter 6 angegebene Restdosis geringer als die maximal mit der Antriebs- und Dosiervorrichtung einstellbare Dosis, gerät der Stoppgegenanschlag 310 in einen Kontakt mit dem Stoppanschlag 210, so dass eine Verdrehung des Dosiseinstellglieds 2 oder des Antriebsglieds 4 relativ zu dem Rotationsglied 7 in eine Drehrichtung, die eine Erhöhung der Dosis zur Folge hätte, d. h. in eine erste Drehrichtung, blockiert wird.

Ein Dosisanzeigeelement 10 weist ein Außengewinde 10b auf, welches in ein Innengewinde 11a des Gehäuses 1 eingreift. Das Dosisanzeigeelement 10, welches hülsenförmig ist und somit als Dosisanzeigetrommel bezeichnet werden kann, weist über seinen Außenumfang eine sich entsprechend der Steigung des Gewindes 10b wendelförmige erstreckenden Dosisskala auf, die eine Vielzahl nacheinander angeordnetes Skalenwerte umfasst. Beispielsweise kann mit der Antriebs- und Dosiervorrichtung eine maximale Dosis von 80 IU eingestellt werden, wobei die Skala von 0 bis 80 und die Dosiswerte in Zweierschritten angegeben sind.

Das Dosisanzeigeelement 10 weist eine in Umfangsrichtung weisende und wirkende Anschlagfläche auf, die als Nulldosisanschlag bezeichnet wird. Das Dosisanzeigeelement weist eine weitere in Umfangsrichtung weisende und wirkende Anschlagfläche auf, welche als Maximaldosisanschlag bezeichnet wird.

Das Dosisanzeigeelement 10 weist ein Außengewinde 10b auf, welches in einem Eingriff mit einem Innengewinde 11a der Außenhülse 1g des Gehäuses 1, oder allgemein des Gehäuses 1 ist. Das Gehäuse 1, insbesondere die Außenhülse 1g weist einen Nulldosisgegenanschlag und einen Maximaldosisgegenanschlag auf.

Das Dosisanzeigeelement 10 kann zwischen einer Nulldosisposition und einer Maximaldosisposition hin und her schraubbar sein. In der Nulldosisposition verhindert der Nulldosisanschlag im Zusammenwirken mit dem Nulldosisgegenanschlag die Drehung des Dosisanzeigeelements 10 in eine zweite Drehrichtung, nämlich in eine Drehrichtung, die bewirken würde, dass eine kleinere Dosis als Null eingestellt wird. In dieser Nulldosisposition ist das Dosisanzeigeelement 10 in die entgegengesetzte, d. h. erste Drehrichtung drehbar.

In der Maximaldosisposition, verhindert der Maximaldosisanschlag im Zusammenwirken mit dem Maximaldosisgegenanschlag die Drehung des Dosisanzeigeelements 10 in die erste Drehrichtung, welche eine Erhöhung der Dosis über den maximal einstellbaren Wert hinaus bewirken würde. Die Drehung in die zweite Drehrichtung ist in der Maximaldosisposition möglich.

Das Gehäuse 1, insbesondere die Außenhülse 1g weist eine Zeigeeinrichtung in der Gestalt eines Fensters auf, welches den Blick auf die Dosisskala des Dosisanzeigeelements 10 freigibt. Das Dosisanzeigeelement 10 ist mit dem Antriebsglied 4 drehfest und axial verschiebbar verbunden. Hierfür weist das Dosisanzeigeelement 10 mindestens eine, in diesem Beispiel mehrere Führungsnuten 10c auf, die sich parallel zur Hauptdrehachse H erstrecken. In diese Führungsnuten 10c greifen Abragungen 4f am Außenumfang des Antriebsglieds 4 ein.

Die Figuren 2 und 3 zeigen einen Auslieferungszustand der Vorrichtung, in dem das Betätigungsglied 8 unbetätigt ist. In der Zeigeeinrichtung erscheint die Dosis null, d. h., dass sich das Dosisanzeigeelement 10 in seiner Nulldosisposition befindet.

Die Antriebsfeder 5 ist mit so viel Energie vorgespannt, dass die in dem Produktbehälter 6 enthaltene Produktmenge mit der in der Feder 5 gespeicherten Energie durch Verschieben des Kolbens 6b vollständig ausgeschüttet werden kann, insbesondere in einer oder in mehreren Einzelausschüttungen, zwischen denen jeweils eine neue Dosiseinstellung stattfindet, ohne die Feder 5 zu spannen. Im Auslieferungszustand sind die erste Kupplung 4a, 7a ausgekuppelt, die zweite Kupplung 2b, 4b eingekuppelt und die dritte Kupplung 1c, 8c ausgekuppelt. Die Antriebsfeder 5 ist kinematisch zwischen das Antriebsglied 4 und das Dosiseinstellglied 2 geschaltet. Die zweite Kupplung 2b, 4b verhindert, dass das Antriebsglied 4 relativ zu dem Dosiseinstellglied 2 gedreht wird.

Zum Einstellen bzw. Erhöhen der auszuschüttenden Produktdosis wird das Dosiseinstellglied 2 relativ zu dem Gehäuse 1 in eine erste Drehrichtung gedreht, wodurch das Antriebsglied 4, die Feder 5 und das Dosisanzeigeelement 10 ebenfalls mitgedreht werden. Durch die Drehung wird das Dosisanzeigeelement 10 von dem Nulldosisgegenanschlag weggeschraubt, wobei der zwischen dem Nulldosisanschlag und dem Nulldosisgegenanschlag entlang der Schraubkurve gemessene Abstand proportional zu der auszuschüttenden Produktdosis ist. Durch die Zeigeeinrichtung 1d kann die aktuell eingestellte Dosis in IU abgelesen werden. Sollte die Dosis versehentlich zu hoch eingestellt worden sein, kann das Dosiseinstellglied 2 durch Drehen in die entgegengesetzte Drehrichtung, d. h. in die zweite Drehrichtung relativ zu dem Gehäuse 1 gedreht werden, wodurch der Abstand zwischen dem Nulldosisanschlag und dem Maximaldosisanschlag abnimmt und die eingestellte Dosis verringert wird.

Für die Produktausschüttung wird das Betätigungsglied 8 zum Beispiel mit dem Daumen der Hand, welche das Gehäuse 1 umgreift, aus einer unbetätigten Position in eine betätigte Position gedrückt, wodurch die Rücksetzfeder 9 gespannt wird. Während der Bewegung des Betätigungsglieds 8 aus der unbetätigten Position in die betätigte Position wird zunächst die dritte Kupplung 1c, 8c eingekuppelt, so dass die Antriebsfeder 5 kinematisch zwischen das Gehäuse 1 und das Antriebsglied 4 geschaltet ist. Das Dosiseinstellglied 2 ist dann auch verdrehgesichert in Bezug auf das Gehäuse 1. Wird das Betätigungsglied 8 weiter in die distale Richtung zu der betätigten Position gedrückt, wird die erste Kupplung 4a, 7a eingekuppelt, wodurch das Rotationsglied 7 in Bezug auf das Antriebsglied 4 verdrehfest ist. Weiteres Verschieben des Betätigungsglieds 8 zu seiner betätigten Position hin bewirkt, dass die zweite Kupplung 2b, 4b ausgekuppelt wird, so dass das Drehmoment der Antriebsfeder 5 über die vierte Kupplung 7a, 11f in das Gehäuse 1 geleitet wird, wodurch sich die Antriebsfeder 5 noch nicht entspannen kann. Erst, wenn die vierte Kupplung 7a, 11f mit dem Erreichen der betätigten Position des Betätigungsglieds 8 ausgekuppelt wird, kann die Antriebsfeder 5, die sich mit ihrem ersten Ende an dem Gehäuse 1 abstützt, die in ihr gespeicherte Federenergie in Form von Rotationsenergie über das Antriebsglied 4 an das Rotationsglied 7 abgegeben, wodurch sich das Antriebsglied 4 in die zweite Drehrichtung dreht. Dabei dreht sich auch das Rotationsglied 7 in die zweite Drehrichtung, wodurch das Abtriebsglied 3 ebenfalls in die zweite Drehrichtung gedreht wird und sich dadurch an dem Innengewinde 1a in die distale Richtung schraubt, wodurch der Kolben 6e in die distale Richtung bewegt wird und das in dem Produktbehälter 6 enthaltene Produkt ausschüttet. Aufgrund der drehfesten Verbindung zwischen dem Antriebsglied 4 und dem Dosiseinstellglied 10 wird gleichzeitig das Dosisanzeigeelement 10 in seine Nulldosisposition zurückgeschraubt. Wenn der Nulldosisanschlag an dem Nulldosisgegenanschlag anschlägt, ist die eingestellte Dosis vollständig ausgeschüttet, wobei durch das Anschlagen des Nulldosisanschlags an dem Nulldosisgegenanschlag das Dosisanzeigeelement 10 hinsichtlich seiner Drehung gestoppt wird, wodurch auch das Antriebsglied 4 hinsichtlich seiner Drehung in die zweite Drehrichtung gestoppt wird. Wenn der Benutzer das Betätigungsglied 8 los lässt, setzt die Rücksetzfeder 9 das Betätigungsglied 8 in seine unbetätigte Position zurück, wobei die erste Kupplung 4a, 7a, die zweite Kupplung 2b, 4b, die dritte Kupplung 1c, 8c und die vierte Kupplung 7a, 11f in ihre Ausgangslagen zurückgestellt werden. Durch Drehen des Dosiseinstellglieds 2 in die erste Drehrichtung kann nun wieder eine auszuschüttende Dosis eingestellt werden, die wieder durch Betätigen des Betätigungsglieds 8 ausgeschüttet werden kann, usw. Wenn in dem Produktbehälter 6 eine Produktmenge enthalten sein sollte, die geringer als die maximal mit der Vorrichtung ausschüttbaren Dosis, in diesem Beispiel kleiner als 80 IU, ist, schlägt der Stoppgegenanschlag 310 an dem Stoppanschlag 210 an, wenn das Dosiseinstellglied 2 in die erste Drehrichtung gedreht wird, bevor der Maximaldosisanschlag an dem Maximaldosisgegenanschlag anschlägt. Hierdurch wird verhindert, dass sich der Verwender der Vorrichtung weniger Produkt injizieren kann als er mit der Vorrichtung eingestellt hat.

In der in den Figuren 1 bis 4 dargestellten ersten Ausführungsform weist das zweite Element 200 eine zweite Gleitfläche 230 auf, die sich insbesondere konzentrisch um eine Nebendrehachse N erstreckt. Die zweite Gleitfläche 230 ist eine Außenumfangsfläche des hülsenförmigen zweiten Elements 200. Das hülsenförmige Stoppelement 300 weist eine erste Gleitfläche 330 auf, welche eine Innenumfangsfläche ist. Die erste Gleitfläche 330 kann drehend an der zweiten Gleitfläche 230 abgleiten. Anders ausgedrückt, ist das hülsenförmige Stoppelement 300 mit seiner ersten Gleitfläche 330 um die Nebendrehachse N drehbar an der zweiten Gleitfläche 230 gelagert. Die Nebendrehachse N ist exzentrisch, d. h. parallel und beabstandet zu der Hauptdrehachse H angeordnet. Das zweite Element 200 ist während der Produktausschüttung um die Hauptdrehachse H drehbar, wobei die exzentrisch dazu angeordneten Nebendrehachse N ebenfalls um die Hauptdrehachse H rotiert.

Das hülsenförmige, das zweite Element 200 umgebende Stoppelement 300 weist über seinen Außenumfang eine erste Eingriffsstruktur 320 auf, die als Außenverzahnung ausgestaltet ist. Die erste Eingriffsstruktur 320 umfasst einen ersten Abschnitt 321 und einen zweiten Abschnitt 322, die entlang der Nebendrehachse N zueinander versetzt, d. h. beabstandet sind. Der Bereich zwischen dem ersten und zweiten Abschnitt 321, 322 kann ohne die erste Eingriffsstruktur 320 auskommen. Das Stoppelement 300 weist einen Stoppgegenanschlag 310 auf, der eine Stoppgegenanschlagfläche bildet, die vorzugsweise in Umfangsrichtung weist. Der Stoppgegenanschlag 310 ist zwischen dem ersten Abschnitt 321 und dem zweiten Abschnitt 322 quer zur Umfangsrichtung federnd angeordnet. Der Stoppgegenanschlag 310 ist an einer elastischen Zunge 323, die sich zum Beispiel in Umfangsrichtung erstreckt integral mit dem Stoppelement 300 gebildet. Der Stoppgegenanschlag 310 kann somit zu der Nebendrehachse N hin und von der Nebendrehachse N wegbewegt werden.

Die erste Eingriffsstruktur 320 erstreckt sich insbesondere konzentrisch um die Nebendrehachse N. Das erste, hülsenförmige Element 100 weist an seinem Innenumfang eine zweite Eingriffsstruktur 120 auf, welche als Innenverzahnung ausgestaltet ist und sich konzentrisch um die Hauptdrehachse H erstreckt. Während der Dosiseinstellung ist das erste Element 100 um die Hauptdrehachse H relativ zu dem zweiten Element 200 drehbar. Während der Dosisausschüttung ist das erste Element 100 zusammen mit dem zweiten Element 200 um die Hauptdrehachse H drehbar. Das erste hülsenförmige Element 100 umgibt das Stoppelement 300 und zumindest die zweite Gleitfläche 230 des zweiten Elements. Zwischen dem ersten Element 100 und dem zweiten Element 200 ist somit ein Ringspalt gebildet, in dem das Stoppelement 300 angeordnet ist, wie am besten aus den Figuren 2 und 3 ersichtlich ist. Dadurch, dass das Stoppelement 300 hülsenförmig ist, wird vorteilhaft erreicht, dass das Stoppelement 300 das Abtriebsglied 3 bzw. die Gewindestange 3a, die oftmals auch als Kolbenstange bezeichnet wird, umgeben kann. Hierdurch wird eine platzsparende Anordnung erreicht.

Durch die exzentrische Anordnung des Stoppelements 300 in Bezug auf die Hauptdrehachse H wird vorteilhaft erreicht, dass die Eingriffsstruktur 320 nur in einem kleinen Bereich des Umfangs mit der zweiten Eingriffsstruktur 120 in einem formschlüssigen Wirkeingriff ist, d. h. kämt. Konstruktiv ist das die Stelle, an der sich die Wälzkreise der ersten Eingriffsstruktur 320 und der zweiten Eingriffsstruktur 120 berühren. Der Wälzkreisdurchmesser der ersten Eingriffsstruktur 320 ist kleiner als der Wälzkreisdurchmesser der zweiten Eingriffsstruktur 120. Über den größten Teil des Umfangs sind die Eingriffsstrukturen 120, 320 außerhalb des Wirkeingriffs. Dadurch, dass die Eingriffsstrukturen 120, 320 im Eingriffsbereich formschlüssig ineinandergreifen, dreht sich das Stoppelement 300 mit der gleichen Umfangsgeschwindigkeit wie das erste Element 100, wobei sich das Stoppelement 300 während der Dosiseinstellung mit einer anderen, insbesondere größeren Winkelgeschwindigkeit dreht als das erste Element 100.

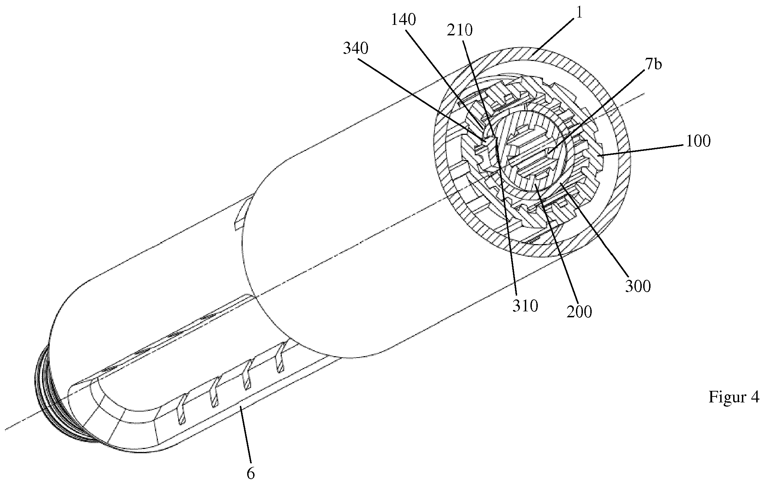

Das zweite Element 200 weist einen Stoppanschlag 210 auf, der eine Stoppanschlagfläche bildet, die vorzugsweise in Umfangsrichtung weist. Die Stoppanschlagfläche mündet an die zweite Gleitfläche 230. Der Stoppanschlagfläche ist in Umfangsrichtung eine Vertiefung in der Gleitfläche 230 vorgelagert. An seinem Innenumfang weist das erste Element 100 eine Schaltstruktur 140 in Gestalt einer Abragung auf. Das Stoppelement 300, insbesondere der Stoppgegenanschlag 310 weist eine Gegenschaltstruktur 340 in der Form einer nach außen abragenden Abragung auf. Die Schaltstruktur 140 und die Schaltgegenstruktur 340 werden während der Dosiseinstellung jeweils auf einer kreisförmigen Bahnkurve bewegt. Jedes aus Schaltstruktur und Schaltgegenstruktur kann seine geschlossene Bahnkurve mehrfach durchlaufen. Durch die unterschiedlichen Winkelgeschwindigkeiten kommen die Schaltstruktur 140 und die Schaltgegenstruktur 340 in einen Anschlag, wodurch die Schaltstruktur 140 die Schaltgegenstruktur 340 und somit auch den Stoppgegenanschlag 310 quer zur Umfangsrichtung und insbesondere zur Nebendrehachse N hin auslenkt, insbesondere in die Ausnehmung, die der Stoppanschlagfläche vorgelagert ist. Dadurch liegen der Stoppanschlag 210 und der Stoppgegenanschlag 310 in Umfangsrichtung gegenüber, so dass der Stoppgegenanschlag 310 gegen den Stoppanschlag 210 gedrückt werden kann, wodurch eine Drehung des Stoppelements 300 in die erste Drehrichtung verhindert wird (Figuren 3 und 4).

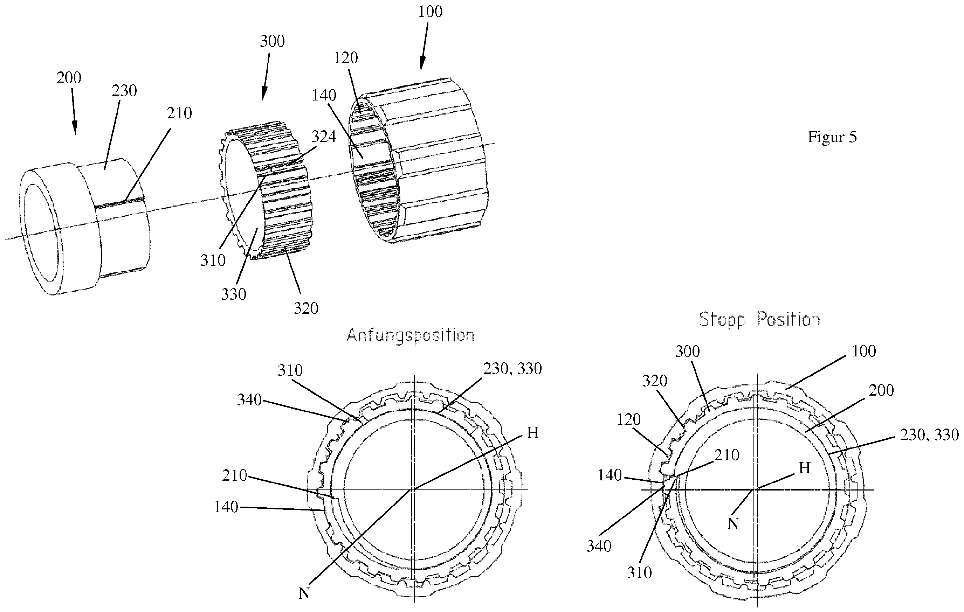

Die zweite Ausführungsform, die in Figur 5 gezeigt wird, ähnelt vom Prinzip her der ersten Ausführungsform, wobei sich das Stoppelement 300 im Wesentlichen durch seine Form unterscheidet. Zum Beispiel kann das erste Element 100 dem in Figur 2 gezeigten Dosiseinstellglied 2 entsprechen, wobei das zweite Element 200 zum Beispiel das Gehäuse 1 sein kann. Andere Positionen für das Stoppelement 300 in einer Injektionsvorrichtung sind selbstverständlich auch denkbar. Das Stoppelement 300 ist eine geschlitzte Hülse. Es weist einen Schlitz 324 auf, der sich über die gesamte Länge des Stoppelements 300, d. h. vom distalen zum proximalen Ende durchgehend erstreckt. Der Schlitz kann schräg oder parallel zur Haupt- oder Nebendrehachse H, N angeordnet sein. Der Schlitz 324 wird beidseitig von jeweils einer Wand des Stoppelements 300 eingefasst. Eine der Wände bildet den Stoppgegenanschlag 310. Das zweite Element 200 weist einen Abschnitt auf, der eine zweite Gleitfläche 230, die als Außenumfangsfläche gebildet ist und konzentrisch um die Nebendrehachse N angeordnet ist, auf. Das Stoppelement 300 weist eine erste Gleitfläche 330 auf, die als Innenumfangsfläche gebildet und konzentrisch um die Nebendrehachse N angeordnet ist. Das Stoppelement 300 ist mit seiner ersten Gleitfläche 330 um die Nebendrehachse N drehbar an der zweiten Gleitfläche 230 gelagert, d. h. exzentrisch zur Hauptdrehachse H.

Das Stoppelement 300 weist eine erste Eingriffsstruktur 320 auf, die sich über den Außenumfang des Stoppelements 300 konzentrisch um die Nebendrehachse N erstreckt und insbesondere als Verzahnung ausgestaltet ist.

Das erste hülsenförmige Element 100 weist an seinem Innenumfang eine zweite Eingriffsstruktur 120 auf, die sich konzentrisch um die Hauptdrehasche H erstreckt, und insbesondere als Verzahnung gebildet ist. Das erste Element 100 kann zum Beispiel konzentrisch, insbesondere drehbar und axialfest an einem Abschnitt des zweiten Elements drehbar gelagert sein, der konzentrisch zur Hauptdrehachse H angeordnet ist. Das zweite Element 200 weist einen Stoppanschlag 210 mit einer in Umfangsrichtung weisenden Stoppanschlagsfläche auf, welcher eine Ausnehmung in der zweiten Gleitfläche 230 vorgelagert ist.

Das erste Element 100 weist an seinem Innenumfang eine Schaltstruktur 140 auf, die zum Beispiel als Abragung gebildet ist. Die Schaltstruktur 140 kann zum Beispiel dadurch gebildet sein, dass der Zwischenraum zwischen zwei Zähnen der Verzahnung aufgefüllt ist.

Das Stoppelement 300 weist an seinem Außenumfang eine Schaltgegenstruktur 340 auf, welche zum Beispiel als Abragung gebildet ist. Die Schaltgegenstruktur 340 ist im Bereich der ersten Eingriffsstruktur 320 angeordnet, wobei die Schaltstruktur 140 im Bereich der zweiten Eingriffsstruktur 120 angeordnet ist. Während der Dosiseinstellung, insbesondere während der Drehung des ersten Elements 100 relativ zu dem zweiten Element 200 in die erste Drehrichtung können die Schaltstruktur 340 und die Schaltgegenstruktur 140 ihre jeweilige kreisförmige Bahnkurve, insbesondere mehrfach durchlaufen bis sie schließlich aneinander anschlagen, wie zum Beispiel aus der Darstellung, in der die Stoppposition in Figur 5 gezeigt wird, ersichtlich ist, wodurch die Schaltgegenstruktur 340 quer zur Umfangsrichtung zur Nebendrehachse N ausgelenkt wird. Dadurch wird auch der Stoppgegenanschlag 310 mit seiner Stoppgegenanschlagfläche quer zur Umfangsrichtung zur Nebendrehachse N hin ausgelenkt. Die Schaltstruktur 140 drückt die Schaltgegenstruktur quer zur Umfangsrichtung in die Ausnehmung, die dem Stoppanschlag 210 vorgelagert ist. Der Stoppanschlag 210 und der Stoppgegenanschlag 310 liegen somit gegenüber, wodurch der Stoppgegenanschlag 310 gegen den Stoppanschlag 210 gedrückt wird, wodurch eine Drehung des ersten Elements 100 relativ zu dem zweiten Element 200 in die erste Drehrichtung verhindert wird.

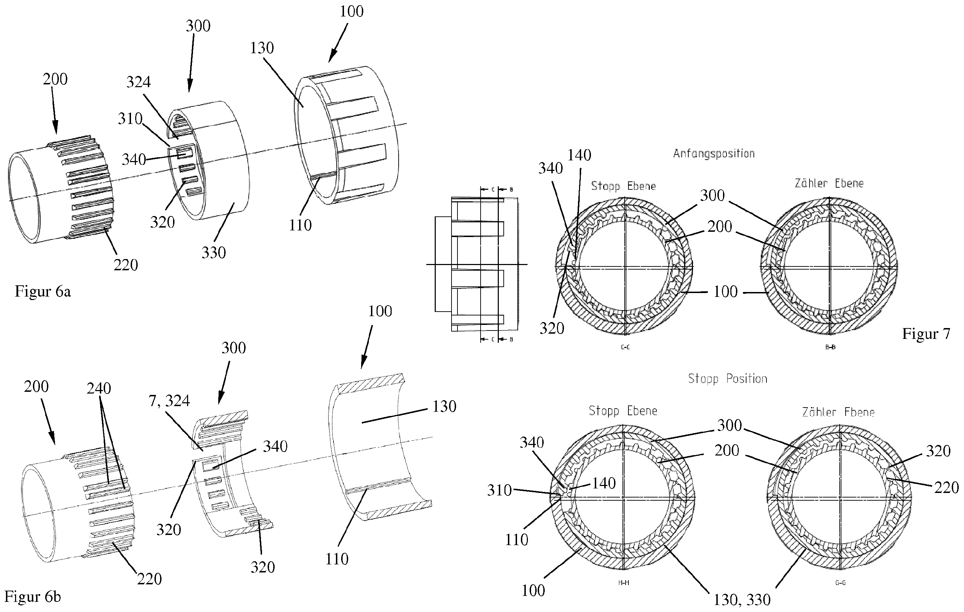

In der in den Figuren 6a, 6b und 7 gezeigten dritten Ausführungsform kann das erste Element 100 zum Beispiel dem Dosiseinstellglied 2 aus Figur 2 und das zweite Element 200 zum Beispiel dem Gehäuse 1 entsprechen, wobei die Ausführungsform nicht hierauf beschränkt ist.

Das zweite Element 200 ist hülsenförmig und weist über seinen Außenumfang eine zweite Eingriffsstruktur 220 in der Gestalt einer Außenverzahnung auf. Die zweite Eingriffsstruktur 220 ist konzentrisch um die Hauptdrehachse H angeordnet. Das erste Element 100 ist relativ zu dem zweiten Element 200 um die Hauptdrehachse H drehbar, insbesondere an dem zweiten Element 200 axialfest und drehbar gelagert. Das erste Element 100 weist an seinem Innenumfang eine zweite Gleitfläche 130 auf, welche konzentrisch zu der Nebendrehachse N angeordnet ist. Vorzugsweise ist die Außenumfangsfläche des ersten Elements 100 konzentrisch zur Hauptdrehachse H angeordnet.

Das erste Element 100 weist einen Stoppanschlag 110 mit einer Stoppanschlagfläche auf, welche insbesondere in Umfangsrichtung weist. In der zweiten Gleitfläche 130 ist dem Stoppanschlag 110 bzw. der Stoppanschlagfläche in Umfangsrichtung eine Ausnehmung vorgelagert.

Das hülsenförmige Stoppelement 300 weist an seinem Innenumfang eine erste Eingriffsstruktur 320 auf, welche als Innenverzahnung gebildet ist. Die erste Eingriffsstruktur 320 erstreckt sich konzentrisch um die Nebendrehachse N. Das Stoppelement 300 ist mit seiner ersten Gleitfläche 330, die als Außenumfangsfläche gebildet ist, um die Nebendrehachse N drehbar an der zweiten Gleitfläche 130 gelagert.

Das Stoppelement 300 weist an seinem Innenumfang eine Schaltgegenstruktur 340 auf, die vorzugsweise als Abragung gebildet ist. Das zweite Element 200 weist eine Schaltstruktur 240 auf, die an seinem Außenumfang gebildet ist, wobei die Schaltstruktur 240 vorzugsweise als Abragung gebildet ist und in dem speziellen Fall aus zwei Zähnen besteht, die im Vergleich zu den Zahnlücken der übrigen Verzahnung einen geringeren Abstand aufweisen.

Das Stoppelement 300 weist einen ersten Abschnitt der sich durchgehend über den Umfang erstreckt, und einen davon separaten zweiten Abschnitt, welcher den Stoppgegenanschlag 320 und die Schaltgegenstruktur 340 bildet, auf. Die Abschnitte sind entlang der Nebendrehachse N versetz zueinander angeordnet. Das Stoppelement 300 weist einen Schlitz 324 auf, der sich nur zum Teil der Gesamtlänge des Stoppelements 300 erstreckt. Der Schlitz 324 ermöglicht, dass der Stoppgegenanschlag 320 quer zur Umfangsrichtung elastisch auslenkbar ist. Der Stoppgegenanschlag 320 ist an einem federnden Arm gebildet, der es ermöglicht, den Stoppgegenanschlag 320 quer zur Umfangsrichtung auszulenken. An dem Arm sind der Stoppgegenanschlag 320 und die Schaltgegenstruktur 340 gebildet. Eine der Wände, welche den Schlitz 324 einfasst, bildet den Stoppgegenanschlag 320.

Während der Dosiseinstellung wird das erste Element 100 relativ zum zweiten Element 200 in die erste Drehrichtung um die Hauptdrehachse H gedreht. Dabei rotiert auch die exzentrisch zur Hauptdrehachse H angeordnete Gleitfläche 130 um die Hauptdrehachse, wodurch die Nebendrehachse N ebenfalls um die Hauptdrehachse H rotiert. Dabei wälzt das Stoppelement 300 in die zweite Drehrichtung auf dem zweiten Element 200 ab. Wenn die Schaltgegenstruktur 340 im weiteren Verlauf an der Schaltstruktur 140 anschlägt bzw. in einen Kontakt kommt (siehe Stopppositionen in Figur 7), wird die Schaltgegenstruktur 340 einschließlich Stoppgegenanschlag 310 von der Schaltstruktur 140 quer zur Umfangsrichtung nach au-βen, d. h. von der Nebendrehachse N weg ausgelenkt. Hierdurch liegen der Stoppgegenanschlag 310 und der Stoppanschlag 110 in Umfangsrichtung gegenüber, wodurch der Stoppgegenanschlag 310 an dem Stoppanschlag 110 anschlagen kann und die Drehung des ersten Elements 100 relativ zu dem zweiten Element 200 in die erste Drehrichtung verhindert.

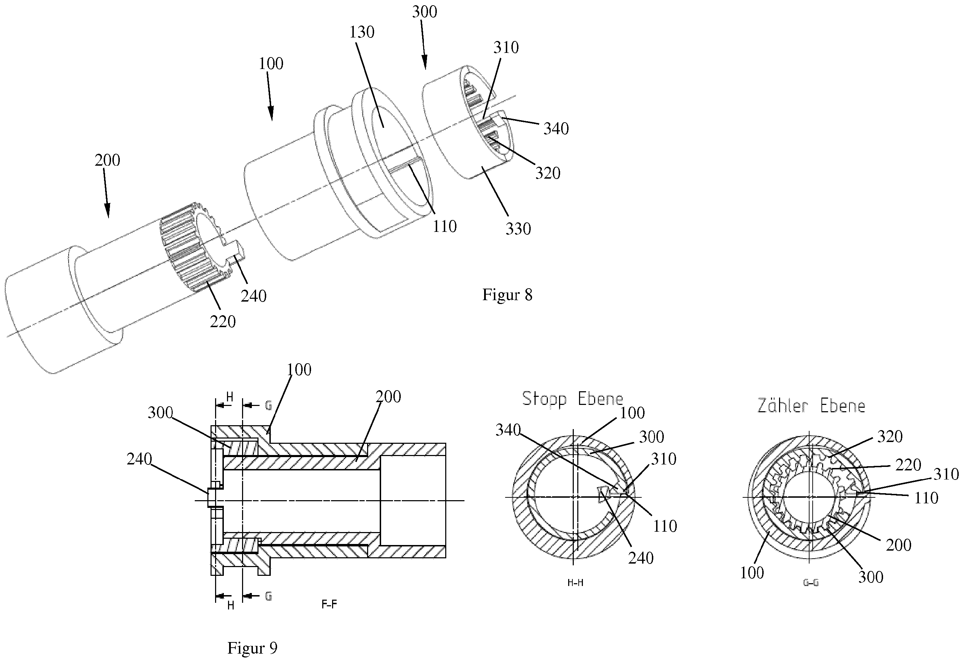

Die vierte Ausführungsform aus den Figuren 8 und 9 funktioniert ähnlich wie die dritte Ausführungsform, weshalb nur die Unterschiede erklärt werden. Das Stoppelement 300 weist einen durchgehenden Schlitz auf. Die Schaltgegenstruktur 340 ist in der Gestalt einer von der ersten Eingriffsstruktur 320 entlang der Nebendrehachse N abgesetzten ersten Abragung. Das zweite Element 200 weist eine von der zweiten Eingriffsstruktur 220 abgesetzte Schaltstruktur 240 auf, in der Gestalt einer Abragung, die sich von der Stirnseite des zweiten Elements 200 in Richtung Nebendrehachse N erstreckt. Die Ausführung aus Figur 9 weist somit eine Stoppebene und eine Zählerebene auf, wobei in der Zählerebene die erste Eingriffsstruktur 320 und die zweite Eingriffsstruktur 220 angeordnet sind und wobei in der Stoppebene die Schaltstruktur 240 und die Schaltgegenstruktur 340 angeordnet sind. Die Stoppanschlagfläche des Stoppanschlags 110 und die Stoppgegenanschlagsfläche des Stoppgegenanschlags 310 können sich in der Stoppebene und/oder in der Zählerebene befinden.

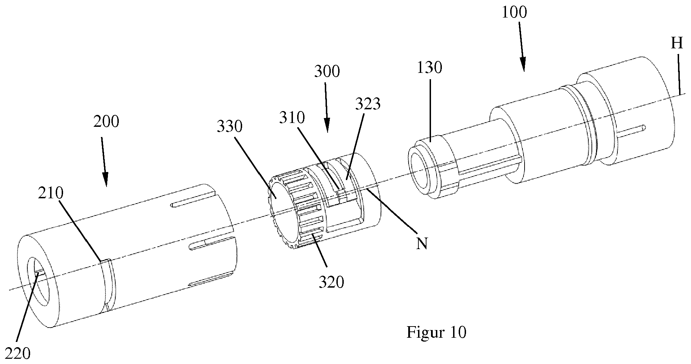

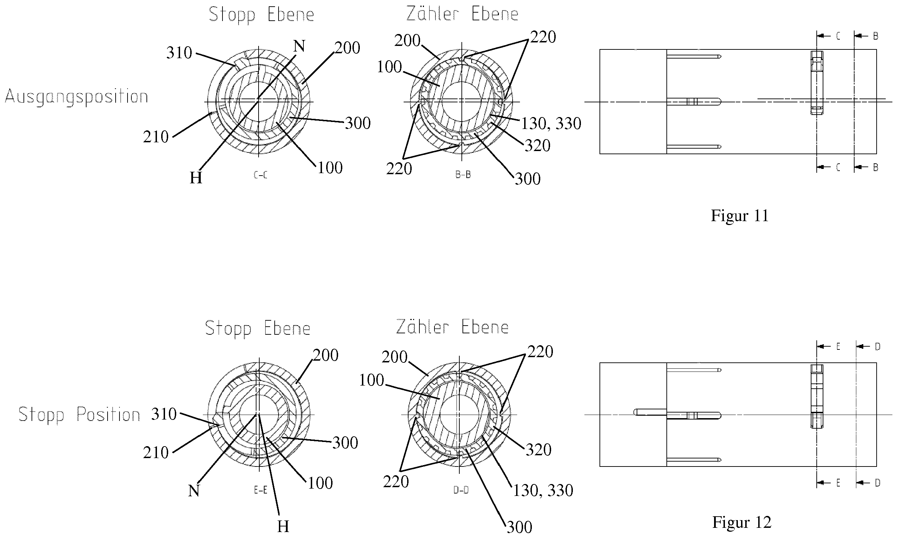

Die in den Figuren 10 bis 12 dargestellte fünfte Ausführungsform weist ein hülsenförmiges zweites Element 200 auf, welches eine zweite Eingriffsstruktur 220 in der Gestalt mehrerer über den Umfang gleichmäßig oder ungleichmäßig verteilter Zähne aufweist (Figuren 11 und 12). Der Abstand zwischen benachbarten Zähnen der zweiten Eingriffsstruktur 220 ist größer als der Abstand zwischen benachbarten Zähnen der ersten Eingriffsstruktur 320 des Stoppelements 300, welches ebenfalls hülsenförmig ausgestaltet ist.

Die zweite Eingriffsstruktur 220 erstreckt sich konzentrisch um die Hauptdrehachse H und ist im Innenumfang des zweiten Elements 200 angeordnet.

Das erste hülsenförmige Element 100 ist über einen ringförmig umlaufender Kragen, der in eine Ringnut des zweiten Elements 200 eingreift, axialfest und um die Hauptdrehachse H drehbar mit dem zweiten Element 200 verbunden. Die zweite Gleitfläche 130 des ersten Elements 100 ist als Außenumfangsfläche gebildet, und erstreckt sich konzentrisch um die Nebendrehachse N. Die Nebendrehachse N ist parallel und beabstandet zur Hauptdrehachse H angeordnet.

Das hülsenförmige Stoppelement 300 weist an seinem Innenumfang eine erste Gleitfläche 330 auf, die sich konzentrisch um die Nebendrehachse N erstreckt und mit der die Stopphülse drehbar an der zweiten Gleitfläche 130 um die Nebendrehachse N gelagert ist. Der Stoppgegenanschlag 310 ist entlang der Nebendrehachse N versetzt zu der ersten Eingriffsstruktur 320 angeordnet. Der Stoppgegenanschlag 310 ist an einer elastischen Zunge 323 gebildet, welche sich in Umfangsrichtung erstreckt. Das zweite Element 200 bildet den Stoppanschlag 210. Der Stoppanschlag 210 wird durch ein Ende einer sich über einen Teil des Umfangs des zweiten Elements erstreckenden Nut gebildet. Im Gegensatz zu den ersten bis vierten Ausführungsformen wird die elastische Zunge 323 nur bei der Montage ausgelenkt, wobei der als Abragung gebildete Stoppgegenanschlag 310 in die sich teilweise über den Umfang erstreckende Nut des zweiten Elements 200 einrastet und dort verbleibt. Die zweite Eingriffsstruktur 320 ist konzentrisch um die Nebendrehachse N angeordnet.

Der Dosiermechanismus weist eine Stoppebene und eine Zählerebene auf, wie aus den Figuren 11 und 12 ersichtlich ist. Die Stoppebene und die Zählerebene stehen jeweils normal auf die Haupt- oder Nebendrehachse H, N. In der Stoppebene sind die sich teilweise über den Umfang erstreckende Nut, der Stoppanschlag 210 und der Stoppgegenanschlag 310 angeordnet. In der Zählerebene, die beabstandet von der Stoppebene ist, sind die erste Eingriffsstruktur 320 und die zweite Eingriffsstruktur 220 angeordnet.

Das Stoppelement 300 ist in dem zwischen dem ersten Element 100 und dem zweiten Element 200 gebildeten Ringspalt angeordnet. Während der Drehung des ersten Elements 100 relativ zu dem zweiten Element 200 um die Hauptdrehachse H, werden die Nebendrehachse N und die konzentrisch dazu angeordnete zweite Gleitfläche 130 um die Hauptdrehachse H in die erste Drehrichtung gedreht, wodurch sich das Stoppelement 300 um die Nebendrehachse N in die entgegengesetzte Drehrichtung, d. h. in die zweite Drehrichtung dreht. Dadurch wird der Stoppgegenanschlag 310 näher zu dem Stoppanschlag 210 gedreht, wie aus dem Vergleich der Ausgangsposition mit der Stoppposition (Figuren 11 und 12 in der Stoppebene) ersichtlich ist. Wenn der Stoppgegenanschlag 310 an dem Stoppanschlag 210 anschlägt, wird auch die Drehung des ersten Elements 100 relativ zu dem zweiten Element 200 in die erste Drehrichtung blockiert.

Wie am besten aus Figur 10 ersichtlich, ist die Gleitfläche 130 ein ringförmig umlaufender Kragen, wobei sich von dem ringförmigen umlaufenden Kragen ein in etwa parallel zur Haupt- oder Nebendrehachse H, N verlaufender Steg erstreckt. Dieser Steg dient als Stabilisierung des Stoppelements 300, damit es nicht quer zur Nebendrehachse N verkippt. Die Lösung mit dem Steg erlaubt eine einfache Montage und Einsparung von Material und hat Vorteile bei der Spritzgussherstellung.

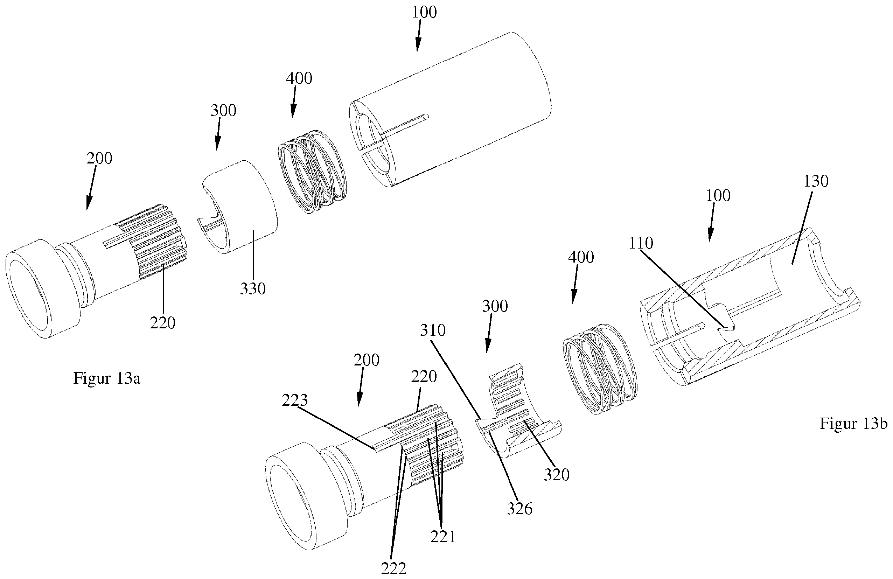

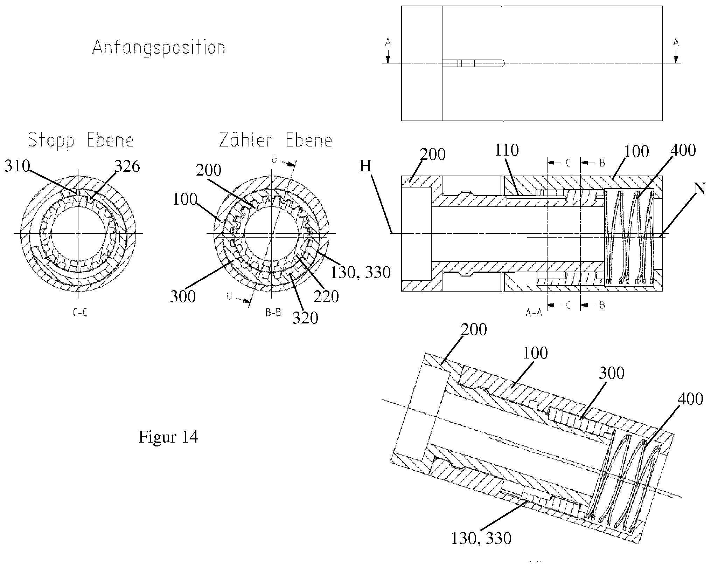

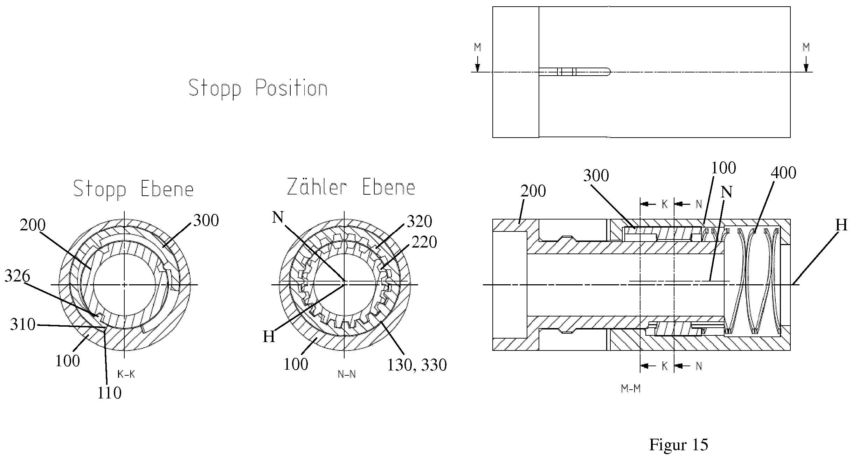

Die sechste Ausführungsform aus den Figuren 13a bis 15 weist ein hülsenförmiges zweites Element 200 und ein hülsenförmiges erstes Element 100 auf, welches über einen Ringkragen, der in eine Ringnut eingreift, axialfest und drehbar mit dem zweiten Element 200 verbunden, insbesondere verschnappt ist. Das erste Element 100 ist relativ zu dem zweiten Element 200 um die Hauptdrehachse H drehbar. Das zweite Element 200 weist eine über seinen Außenumfang angeordnete zweite Eingriffsstruktur 220 auf, die als Außenverzahnung gebildet ist und eine Vielzahl von Führungsbahnen aufweist. Das Stoppelement 300 weist eine über seinen Innenumfang angeordnete erste Eingriffsstruktur 320 auf. Die zweite Eingriffsstruktur 220 ist konzentrisch zur Hauptdrehachse H angeordnet, wobei die erste Eingriffsstruktur 320 konzentrisch um die Nebendrehachse N angeordnet ist, die exzentrisch zur Hauptdrehachse H angeordnet ist. Das hülsenförmige Stoppelement 300 weist über seinen Außenumfang eine erste Gleitfläche 330 auf, die konzentrisch zur Nebendrehachse N angeordnet ist. Das erste hülsenförmige Element 100 weist an seinem Innenumfang eine konzentrisch zur Nebendrehachse N angeordnete zweite Gleitfläche 130 auf, an der das Stoppelement 300 über seine erste Gleitfläche 330 drehbar um die Nebendrehachse N gleitgelagert ist. Eine vorgespannte Feder 400, die vorzugsweise als Druckfeder wirkt, ist zwischen dem ersten Element 100 und dem Stoppelement 300 angeordnet und stützt sich an dem Stoppelement 300 und dem ersten Element 100 ab. Die Feder 400 versucht das hülsenförmige Element 300 aus der in Figur 14 gezeigten Anfangsposition entlang der Nebendrehachse N in eine Stoppposition zu verschieben, in welcher der Stoppgegenanschlag 310 dem Stoppanschlag 110 des ersten Elements 100 in Umfangsrichtung gegenüberliegt. Die Bewegung des Stoppelements 300 entlang der Nebendrehachse N wird erst dann freigegeben, wenn ein Eingriffsglied 326, welches am Innenumfang des Stoppelements 300 gebildet wird in eine bestimmte Führungsbahn der Eingriffsstruktur 220 eingreift. Ist dies nicht der Fall, kann das Stoppelement 300 nicht verschoben werden.

Das Ende 323 mindestens einer der Führungsbahnen 221 ist entlang der Hauptdrehachse H oder Nebendrehachse N versetzt zu den Enden 222 der anderen Führungsbahnen 221 angeordnet. Die mindestens eine Führungsbahn 221, welche das versetzte Ende 223 aufweist, kann für die bessere Unterscheidbarkeit als lange Führungsbahn bezeichnet werden. Die anderen Führungsbahnen 221 mit den Enden 220, können als kurze Führungsbahnen bezeichnet werden. Das Stoppelement 300 weist vorzugsweise mehrere Eingriffsglieder 326 über seinen Umfang verteilt auf, insbesondere so viele, dass immer eines der Eingriffsglieder 326 in einem Eingriff mit den Führungsbahnen 221 ist. Dadurch wird sichergestellt, dass das Stoppelement 300 nur dann axial verschoben werden kann, wenn das Eingriffsglied 326 in der langen Führungsbahn ist. In der Anfangsposition befindet sich das Eingriffsglied 326 in einer kurzen Führungsbahn. Das erste Element 100 kann um die Hauptdrehachse H relativ zu dem zweiten Element 200 in die erste Drehrichtung gedreht werden, wobei das Eingriffsglied 326 auf einer ihm zugeordneten Bahnkurve bewegt wird. Nach einer bestimmten Drehung, insbesondere mehrfachen Umdrehung des ersten Elements 100 um die Hauptdrehachse H gerät das Eingriffsglied 326 in den Eingriff mit der langen Führungsbahn. Solange das Eingriffsglied 326 in einer kurzen Führungsbahn ist, verhindert das Ende 220, dass das Stoppelement 300 axial verschoben wird. Da das Ende 223 der langen Führungsbahn gegenüber den Enden 222 der kurzen Führungsbahnen versetzt ist, kann das Stoppelement 300 axial verschoben werden. Der Stoppgegenanschlag 310 wird somit in eine Stoppebene, in der sich auch der Stoppanschlag 110 befindet, verschoben. Mit anderen Worten liegt der Stoppgegenanschlag 310 dem Stoppanschlag 110 in Umfangsrichtung gegenüber, so dass eine Drehung des ersten Elements 100 relativ zu dem zweiten Element 200 um die erste Drehrichtung den Stoppgegenanschlag 310 gegen den Stoppanschlag 110 drückt, wodurch eine Drehung des ersten Elements 100 relativ zu dem zweiten Element 200 in die erste Drehrichtung verhindert bzw. blockiert wird.

Soll die eingestellte Dosis zum Beispiel durch Zurückdrehen des ersten Elements 100 relativ zu dem zweiten Element 200 in die zweite Drehrichtung korrigiert werden, können das erste Element 100 und das Stoppelement 300 so aufeinander abgestimmt sein, dass eine Drehung das Stoppelement 300 entlang der Nebendrehachse N zurückschiebt und dabei die Feder 400 wieder spannt.

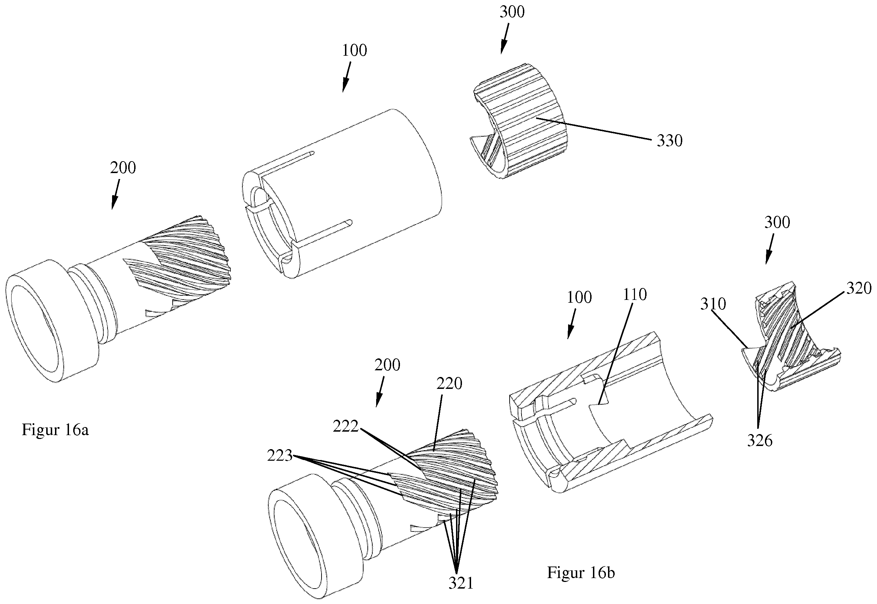

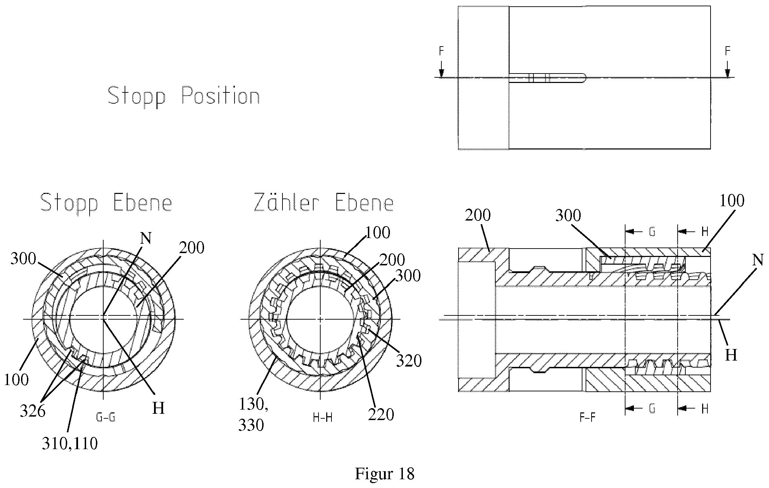

Die siebte Ausführungsform, welche in den Figuren 16a bis 18 dargestellt wird, entspricht prinzipiell der sechsten Ausführungsform, wobei die siebte Ausführungsform ohne Feder 400 auskommt, dafür aber eine zweite, konzentrisch um die Hauptdrehachse H angeordnete Eingriffsstruktur 220 in der Gestalt einer Schrägverzahnung aufweist, welche Führungsbahnen 221, welche helixförmig um die Hauptdrehachse H angeordnet sind, aufweist. Ferner sind die erste Gleitfläche 330 und die zweite Gleitfläche 130, die konzentrisch um die Nebendrehachse N angeordnet sind, so aufeinander abgestimmt, dass die Reibung zwischen dem ersten Element 100 und dem Stoppelement 300 etwas erhöht ist, wodurch das erste Element 100 und das Stoppelement 300 aufgrund des Reibeingriffs mitdrehen kann, wenn das Eingriffsglied 326 in der Führungsbahn 221 ist, deren Ende 223 versetzt angeordnet ist (lange Führungsbahn), und das erste Element 100 in die erste Drehrichtung oder zur Dosiskorrektur in die zweite Drehrichtung gedreht wird.

Die erste Eingriffsstruktur 320 des Stoppelements 300 ist konzentrisch zur Nebendrehachse N über den Innenumfang des Stoppelements 300 angeordnet. An dem Innenumfang ist mindestens ein Eingriffsglied 326, welches prinzipiell die gleiche Funktion hat, wie das Eingriffsglied 326 aus der sechsten Ausführungsform.

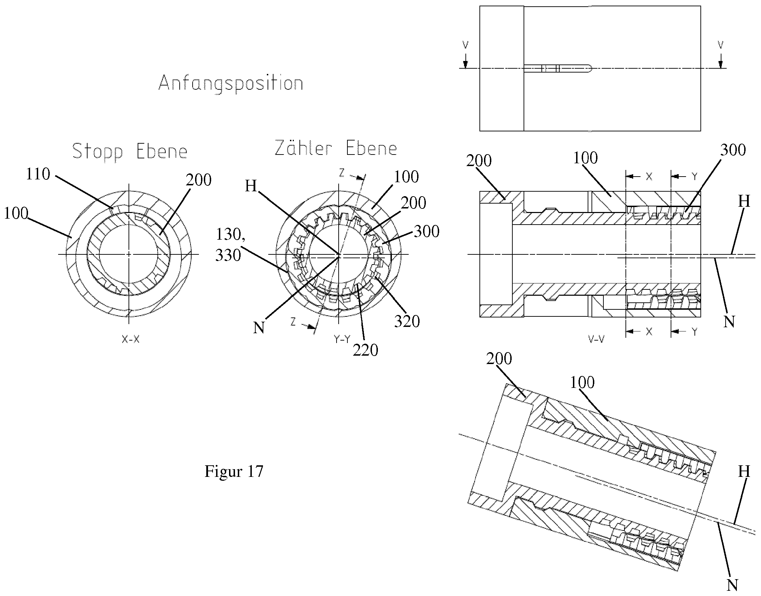

In der Ausgangsposition, wie sie in Figur 17 dargestellt ist, befindet sich das mindestens eine Eingriffsglied 326 in einer kurzen Führungsbahn, d. h. in einer Führungsbahn mit den Enden 222.

Das erste Element 100 kann relativ zu dem zweiten Element 200 um die Hauptdrehachse H in die erste Drehrichtung gedreht werden, wobei mindestens eines der Eingriffsglieder 326 in einer kurzen Führungsbahn ist, wobei das Ende 222 verhindert, dass das Stoppelement 320 entlang der Nebendrehachse N verschoben wird. Solange das Eingriffsglied 326 in einer der kurzen Führungsbahnen ist, kann die zweite Gleitfläche 130 an der ersten Gleitfläche 330 abgleiten. Der Stoppanschlag 110 und der Stoppgegenanschlag 310 befindet sich auf verschiedenen Positionen entlang der Nebendrehachse N, so dass der Stoppgegenanschlag 310 an dem Stoppanschlag 110 mindestens einmal, vorzugsweise mehrfach vorbeibewegt werden kann. Im weiteren Verlauf der Drehung des ersten Elements 100 in die erste Drehrichtung gerät das Eingriffsglied 326 in die lange Führungsbahn, dessen Ende 223 axialversetzt zu den Enden 222 der kurzen Führungsbahnen angeordnet sind. Durch den Reibeingriff der zweiten Gleitfläche 130 mit der ersten Gleitfläche 330 wird das Stoppelement 300 helixförmig entlang der Hauptdrehachse H oder Nebendrehachse N verschoben, d. h. geschraubt. Hierdurch wird der Stoppgegenanschlag 310 in eine Position verschoben, in der er in Umfangsrichtung dem Stoppanschlag 110 gegenüberliegt. Eine weitere Drehung des ersten Elements 100 in die erste Drehrichtung bewirkt, dass der Stoppgegenanschlag 310 gegen den Stoppanschlag 110 gedrückt wird, wodurch eine Drehung des ersten Elements 100 relativ zu dem zweiten Element 200 in die erste Drehrichtung verhindert wird.

Zur Dosiskorrektur kann das erste Element 100 relativ zu dem zweiten Element 200 in die entgegengesetzte, d. h. zweite Drehrichtung gedreht werden, wodurch aufgrund des Reibeingriffs zwischen dem ersten Element 100 und dem Stoppelement 300 das Stoppelement 300 entlang der Haupt- oder Nebendrehachse H, N zurückgeschraubt wird.

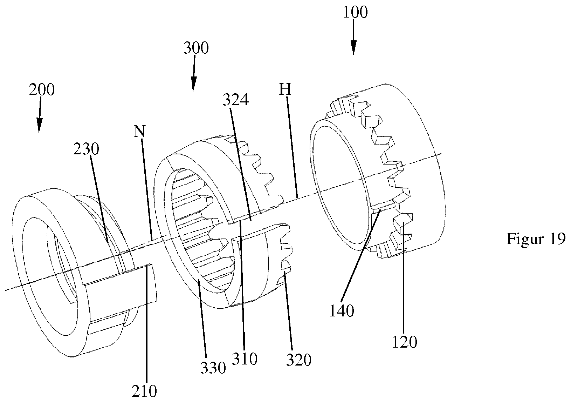

Die in den Figuren 19 bis 21 gezeigte achte Ausführungsform weist ein erstes hülsenförmiges Element 100, ein zweites hülsenförmiges Element 200 und ein hülsenförmiges Stoppelement 300 auf. Das erste Element 100 ist um die Hauptdrehachse H relativ zu dem zweiten Element 200 drehbar, insbesondere axialfest und drehbar mit dem zweiten Element 200 verbunden. Das Stoppelement 300 weist eine erste Gleitfläche 330 auf, die konzentrisch um die Nebendrehachse N, die in Bezug auf die Hauptdrehachse H in einem spitzen Winkel angeordnet ist und die die Hauptdrehachse H vorzugsweise schneidet oder alternativ windschief dazu angeordnet ist, angeordnet ist. Die erste Gleitfläche 330 ist eine Innenumfangsfläche. Die zweite Gleitfläche 230, die von dem zweiten hülsenförmigen Element 200 gebildet wird, ist konzentrisch um die Nebendrehachse N angeordnet und eine Außenumfangsfläche. Das Stoppelement 300 ist mit seiner ersten Gleitfläche 330 um die Nebendrehachse N drehbar an der zweiten Gleitfläche 230 geleitgelagert. Das erste Element 100 weist eine zweite Eingriffsstruktur 120 in der Gestalt einer Stirnverzahnung auf, die zu dem Stoppelement 300 weist. Die zweite Eingriffsstruktur 120 erstreckt sich konzentrisch um die Hauptdrehachse H.

Das Stoppelement 300 weist eine erste Eingriffsstruktur 320 und eine Innenverzahnung auf. Die Eingriffsstruktur 320, insbesondere die Stirnverzahnung kämmt mit der Eingriffsstruktur 120.

Das erste Element 100 weist eine Schaltstruktur 140 auf, die an einem Außenumfang, der der Eingriffsstruktur 120 vorgelagert ist, gebildet ist und radial nach außen abragt. Das Stoppelement 300 weist eine Schaltgegenstruktur 340 auf, die an dem Innenumfang des Stoppelements 300 angeordnet ist.

Das Stoppelement 300 ist eine geschlitzte Hülse, d. h., dass das Stoppelement 300 einen Schlitz 324 aufweist, der sich in dem gezeigten Beispiel über die gesamte Länge des Stoppelements 300 erstreckt. Eine der den Schlitz 324 einfassenden Wände des Stoppelements 300 bildet einen Stoppgegenanschlag 310. An dem Ende mit dem Stoppgegenanschlag 310 ist auch die Schaltgegenstruktur 340 angeordnet.

Die Eingriffsstruktur 120, 320 sind so aufeinander abgestimmt, dass sich das Stoppelement 300 mit einer anderen Winkelgeschwindigkeit, insbesondere größeren Winkelgeschwindigkeit um die Nebendrehachse N dreht, als sich das erste Element 100 um die Hauptdrehachse H dreht. Hierzu kann die erste Eingriffsstruktur 320 eine geringere Zähnezahl aufweisen als die zweite Eingriffsstruktur 120.

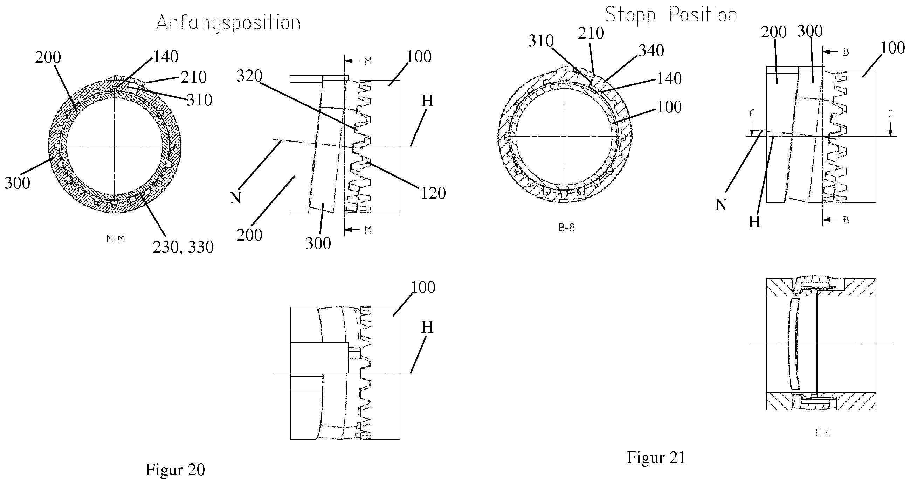

Während der Drehung des ersten Elements 100 relativ zu dem zweiten Element 200 um die Hauptdrehachse H wird die Schaltstruktur 140 auf einer kreisförmigen Bahnkurve um die Hauptdrehachse H bewegt, die sie zum Beispiel mehrfach durchlaufen kann. Ferner wird die Schaltgegenstruktur 340 auf ihrer kreisförmigen Bahnkurve um die Nebendrehachse N bewegt, die sie zum Beispiel mehrfach durchlaufen kann. Aufgrund der unterschiedlichen Winkelgeschwindigkeiten geraten die Schaltstruktur 140 und die Schaltgegenstruktur 340 nach einem bestimmten Gesamtdrehwinkel in einen gegenseitigen Kontakt, wodurch die Schaltstruktur 140 die Schaltgegenstruktur 340 und somit den Stoppgegenanschlag 310 quer zur Umfangsrichtung und von der Nebendrehachse N weg auslenkt, wodurch der Stoppgegenanschlag 310 in eine Position ausgelenkt wird, in der er in Umfangsrichtung dem Stoppanschlag 210, der von dem zweiten Element 200 gebildet wird, gegenüberliegt. Durch Drehen des ersten Elements 100 relativ zu dem zweiten Element 200 in die erste Drehrichtung wird der Stoppgegenanschlag 310 gegen den Stoppanschlag 210 gedrückt, wodurch eine weitere Drehung des ersten Elements 100 relativ zu dem zweiten Element 200 um die Hauptdrehachse H verhindert bzw. blockiert wird (Figur 21).

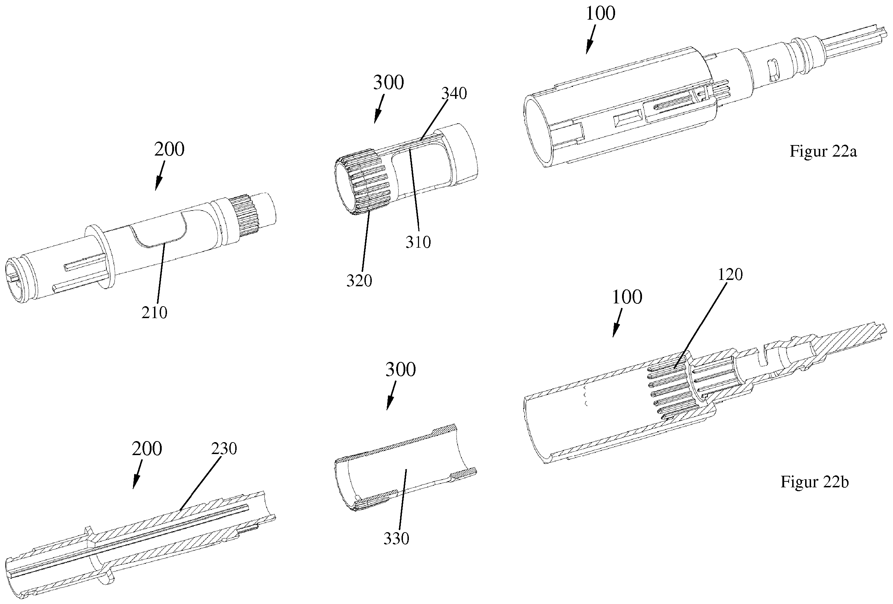

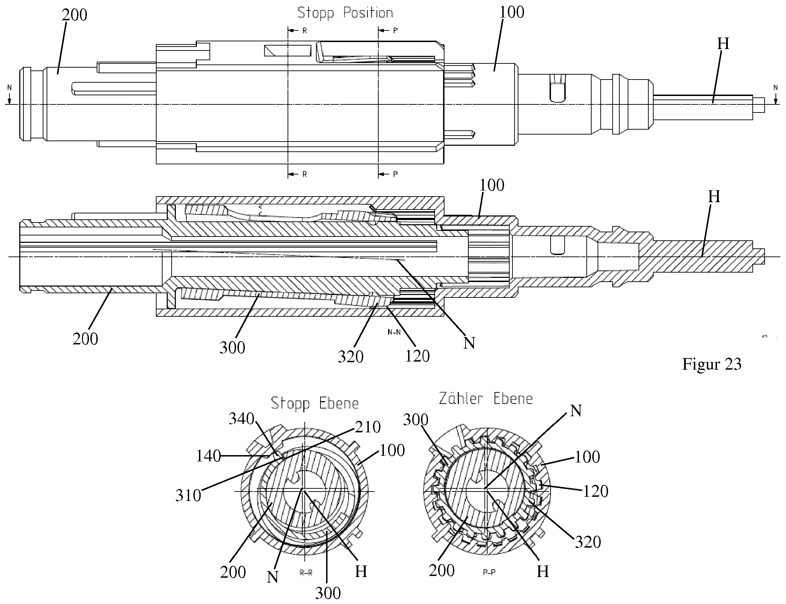

Die in den Figuren 22a bis 23 gezeigte neunte Ausführungsform weist ein hülsenförmiges erstes Element 100 und ein hülsenförmiges zweites Element 200 auf, wobei das hülsenförmige Element 100 und das hülsenförmige Element 200 umgibt. Das hülsenförmige Element 200 kann zum Beispiel in einem direkten verdrehgesicherten und axial verschiebbaren Eingriff mit einer Kolbenstange sein. In dem einen Ringspalt zwischen dem hülsenförmigen ersten Element 100 und dem hülsenförmigen zweiten Element 200 ist ein hülsenförmiges Stoppelement 300 angeordnet, welches um eine Nebendrehachse N drehbar ist. Das erste hülsenförmige Element 100 kann zum Beispiel dem Antriebsglied 4 und das zweite hülsenförmige Element 200 kann zum Beispiel dem Abtriebsglied 3 der Injektionsvorrichtung aus Figuren 2 und 3 entsprechen.

In den Figuren 22a und 22b wird das Stoppelement 300 verkehrtherum dargestellt. Die Eingriffsstruktur 320 müsste in die proximale Richtung weisen, um mit der Eingriffsstruktur 120 in einen Eingriff kommen zu können. Das zweite Element 200 weist eine konzentrisch um die Nebendrehachse N angeordnete Gleitfläche 230 auf, die als Außenumfangsfläche gebildet ist. Das Stoppelement 300 weist eine konzentrisch zur Nebendrehachse angeordnete erste Gleitfläche 330 auf, die als Innenumfangsfläche gebildet ist. Das Stoppelement 300 weist eine erste Eingriffsstruktur 320 in der Gestalt einer Außenverzahnung auf, die konzentrisch zur Nebendrehachse N angeordnet ist. Das erste Element 100 weist an seinem Innenumfang eine zweite Eingriffsstruktur 120 auf, die als Verzahnung gebildet ist und konzentrisch zur Hauptdrehachse H angeordnet ist. Das Stoppelement 300 umgibt das zweite Element 200. Insofern lässt sich diese Ausführungsform mit der ersten Ausführungsform vergleichen.

Das erste Element 100 weist an seinem Innenumfang eine Schaltstruktur 140 auf, wobei das Stoppelement 300 an seinem Außenumfang eine Schaltgegenstruktur 340 aufweist. Jede aus Schaltstruktur 140 und Schaltgegenstruktur 340 kann ihre jeweilige Bahnkurve, vorzugsweise mehrfach durchlaufen, bis die Schaltstruktur 140 und die Schaltgegenstruktur 340 aneinander anschlagen. Wenn die Schaltstruktur 140 und die Schaltgegenstruktur 340 aneinander anschlagen, wird das Stoppelement 300 um eine Kippachse, die quer zur Nebendrehachse N und Hauptdrehachse H angeordnet ist, gekippt. Hierdurch wird auch die Nebendrehachse N in Bezug auf die Hauptdrehachse H gekippt. Da der Stoppgegenanschlag 310 bevorzugt starr an dem Stoppelement 300 angeordnet ist, wird der Stoppgegenanschlag 310 mit dem Stoppelement 300 gekippt.

Das zweite Element 200 weist in seiner zweiten Gleitfläche 230 eine dem Stoppanschlag 210 in Umfangsrichtung vorgelagerte Ausnehmung auf. Wenn das Stoppelement 300 in seine ausgelenkte Position gekippt wird, wird der Stoppgegenanschlag 310 in Umfangsrichtung vor dem Stoppanschlag 210 bewegt, insbesondere gekippt, wodurch der Stoppgegenanschlag 310 an dem Stoppanschlag 210 anschlägt und die Drehung des ersten Elements 100 relativ zu dem zweiten Element 200 in die erste Drehrichtung verhindert.

Die Nebendrehachse N kann in der unausgelenkten Position des Stoppelements 300 oder wenn die Schaltstruktur 140 und die Schaltgegenstruktur 340 nicht aneinander anschlagen, parallel zur Hauptdrehachse H sein oder in Bezug auf die Hauptdrehachse H frei kippbar sein. Kommen dabei und bei Drehung des ersten Elements 100 relativ zu dem zweiten Element 200 in die erste Drehrichtung der Stoppgegenanschlag 310 und der Stoppanschlag 210 in Berührung bewirkt deren Formgebung die Rückstellung des Stoppelements 300 in die unausgelenkte Position. Die Nebendrehachse N wird erst dann zwangsweise in Bezug auf die Hauptdrehachse H gekippt bzw. gekippt gehalten, wenn die Schaltstruktur 140 und die Schaltgegenstruktur 340 aneinander anschlagen. Insbesondere ist zwischen dem ersten Element 100 und dem zweiten Element 200 der Ringspalt hinsichtlich seiner Spaltbreite so bemessen, dass das Stoppelement 300 um die Kippachse kippen kann.

Die Ausführungsformen 1 bis 9 sind in Anlehnung an den ersten hierin beschriebenen Aspekt ausgebildet.

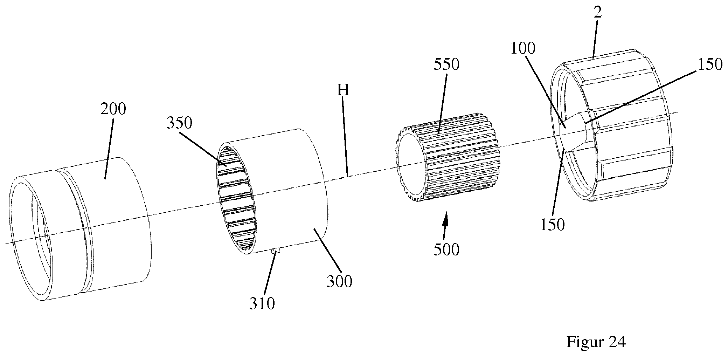

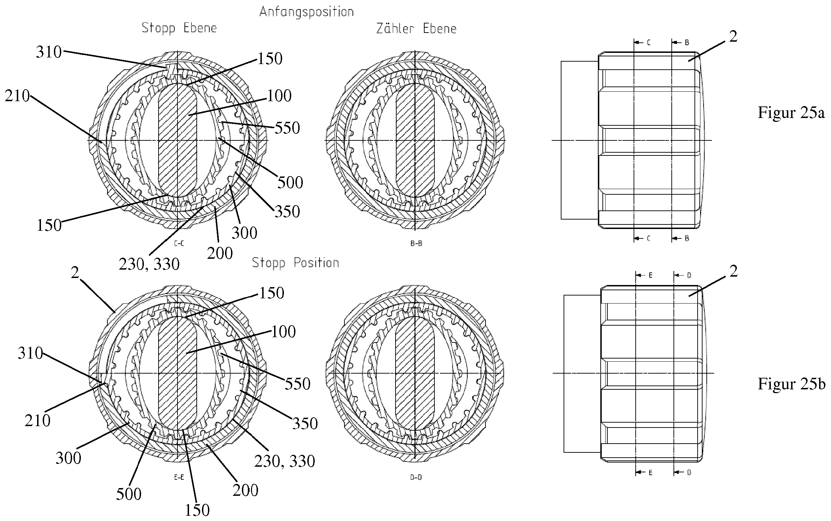

Die in den Figuren 24 bis 25b gezeigte zehnte Ausführungsform, die in Anlehnung an den zweiten hierin beschriebenen Aspekt ausgebildet ist, weist ein erste Element 100, ein zweites hülsenförmiges Element 200, ein hülsenförmiges Stoppelement 300 und eine flexible Hülse 500 auf. Die flexible Hülse 500 ist mit dem zweiten Element 200 verdrehfest verbunden. Das erste Element 100 ist um die Hauptdrehachse H relativ zu dem zweiten Element drehbar. Eine Nebendrehachse ist hier nicht erforderlich. Das erste Element 100 ist über das hülsenförmige Stoppelement 300 und die flexible Hülse 500 gekoppelt. Das erste Element 100 kann zum Beispiel mit einem Dosiseinstellelement 2, welches hülsenförmig ist, gekoppelt, insbesondere verbunden oder einteilig gebildet sein.

Das hülsenförmige Stoppelement 300 weist eine Innenverzahnung 350 auf, die sich insbesondere konzentrisch um die Hauptdrehachse H erstreckt. Das Stoppelement 300 bildet einen Stoppgegenanschlag 310, vorzugsweise an seinem Außenumfang. Die flexible Hülse 500 weist eine Außenverzahnung 550 auf, welche in die Innenverzahnung 350 eingreift bzw. mit der Innenverzahnung 350 kämmt. Das erste Element 100 bildet zwei Gleitflächen 150, welche die flexible Hülse 500 oval aufspannen, insbesondere in etwa elliptisch aufspannen, und die am Innenumfang der flexiblen Hülse 500 abgleiten, wenn das erste Element 100 relativ zu dem zweiten Element 200 in die erste Drehrichtung gedreht wird. Dadurch, dass die Hülse 500 flexibel ist, wird sie bei der Drehung des ersten Elements 100 relativ zu dem zweiten Element 200 verformt. Die Zähnezahl der Außenverzahnung 550 ist vorzugsweise geringer als die Zähnezahl der Innenverzahnung 350.

Das zweite Element 200 bildet eine zweite Gleitfläche 230, die als Innenumfangsfläche gebildet ist. Das Stoppelement 300 bildet eine erste Gleitfläche 330, die als Außenumfangsfläche gebildet ist. Das Stoppelement 300 ist um die Hauptrehachse H drehbar an der zweiten Gleitfläche 230 geleitgelagert. Aufgrund der geringeren Zähnezahl der flexiblen Hülse 500 wird der Stoppgegenanschlag 310 in die zweite Drehrichtung bzw. zu dem zweiten Element 200 gebildeten Stoppanschlag 210 hinbewegt, wenn das erste Element 100 in die erste Drehrichtung gedreht wird.

Wenn der Stoppgegenanschlag 310 seine Stoppposition erreicht, d. h. die Position, an der der Stoppgegenanschlag 310 an dem Stoppanschlag 210 anschlägt bzw. ihn berührt, wird die Drehung der Stopphülse 300 in die zweite Drehrichtung blockiert, wodurch die Drehung des ersten Elements 100 in die erste Drehrichtung ebenfalls blockiert wird, da eine Drehung des ersten Elements 100 in die erste Drehrichtung voraussetzt, dass das Stoppelement 300 in die zweite, der ersten Drehrichtung entgegengesetzte Drehrichtung drehbar ist.

Lediglich beispielhaft sei erwähnt, dass das erste Element 100 dem Dosiseinstellglied 2 (Figuren 2 und 3) entsprechen kann. Das zweite Element 200 kann zum Beispiel dem Gehäuse 1 entsprechen.

**Bezugszeichenliste**

| | | | |
|---|---|---|---|
| 1 | Gehäuse | 11 | Verschiebeglied |
| la | Gewinde / Innengewinde | 11a | Gewinde |
| 1c | fünfte Kupplungsstruktur | 11f | Innenverzahnung / siebte Kupplungsstruktur |
| 1f | Abragung | | |
| 1g | Außenhülse | 12 | Produktbehälterhalter |
| 1h | Innenhülse | 4a, 7a | erste Kupplung |
| 2 | Dosiseinstellglied | 2b, 4b | zweite Kupplung |
| 2a | Abragung | 1c, 8c | dritte Kupplung |
| 2b | dritte Kupplungsstruktur | 7e, 11f | vierte Kupplung |
| | | | |
| 3 | Abtriebsglied | 100 | erstes vzw. hülsenförmiges Element |
| 3a | Gewindestange | 110 | Stoppanschlag |
| 3b | Flansch | 120 | zweite Eingriffsstruktur |
| 3c | Gewinde / Außengewinde | 130 | zweite Gleitfläche |
| 3d | Führung / Führungsnut | 140 | Schaltstruktur |
| | | 150 | Gleitfläche |
| 4 | Antriebsglied | | |
| 4a | erste Kupplungsstruktur | 200 | zweites, vzw. hülsenförmiges Element |
| 4b | vierte Kupplungsstruktur | 210 | Stoppanschlag |
| 4e | Kragen | 220 | zweite Eingriffsstruktur |
| 4f | Abragung / Steg | 221 | Führungsbahn |
| | | 222 | Ende |
| 5 | Antriebsfeder | 223 | Ende |
| | | 230 | zweite Gleitfläche |
| 6 | Produktbehälter / Karpule | 240 | Schaltstruktur, Abragung / Nocke |
| 6a | Behälterkörper | | |
| 6b | Kolben | 300 | hülsenförmiges Stoppelement |
| 6c | Septum | 310 | Stoppgegenanschlag |
| | | 320 | erste Eingriffsstruktur |
| 7 | Rotationsglied | 321 | erster Abschnitt |
| 7a | zweite Kupplungsstruktur | 322 | zweiter Abschnitt |
| 7b | Führung / Eingriffsglied | 323 | elastische Zunge |
| 7c | Rastmittel / Rastarm | 324 | Schlitz |
| 7e | achte Kupplungsstruktur | 326 | Eingriffsglied |
| | | 330 | erste Gleitfläche |
| 8 | Betätigungsglied / Betätigungsknopf | 340 | Schaltgegenstruktur, Nocke / Abragung |
| 8a | Verbindungsglied | 350 | Innenverzahnung |
| 8b | Abdeckkappe | | |
| 8c | sechste Kupplungsstruktur | 400 | Feder |
| 8d | Kontaktfläche | | |
| 8e | Eingriffsnocke | 500 | flexible Hülse |
| | | 550 | Außenverzahnung |
| 9 | Rücksetzfeder | | |
| | | | |
| 10 | Dosisanzeigeelement / Dosisanzeigetrommel | N | Nebendrehachse |
| 10b | Gewinde | H | Hauptdrehachse |
| 10c | Führungsnut | | |

## Patentansprüche

1. Dosiermechanismus für eine Injektionsvorrichtung, umfassend:
a) ein Dosiseinstellelement (2) und ein damit gekoppeltes erstes Element (100), das mittels einer Kupplung während einer Dosiseinstellung relativ zu einem anderen, zweiten Element (200) um eine Hauptdrehachse (H) in eine erste Drehrichtung drehbar und während einer Dosisausschüttung relativ zu dem zweiten Element (200) drehfest ist, wobei das erste Element (100) und das zweite Element (200) über ein hülsenförmiges Stoppelement (300), welches einen Stoppgegenanschlag (310) bildet, gekoppelt sind, und
b) einen Stoppanschlag (110; 210), wobei der Stoppgegenanschlag (310) während der Drehung des ersten Elements (100) in die erste Drehrichtung eine Bewegung zu einer Stoppposition hin ausführt, wobei in der Stoppposition der Stoppgegenanschlag (310) an dem Stoppanschlag (110; 210) anschlägt und die Drehung des ersten Elements (100) relativ zu dem zweiten Element (200) in die erste Drehrichtung verhindert,
**dadurch gekennzeichnet, dass**
c) das hülsenförmige Stoppelement (300) um eine Nebendrehachse (N), die parallel versetzt oder winkelversetzt zur Hauptdrehachse (H) angeordnet ist, drehbar ist und das erste Element (100) oder das zweite Element (200) umgibt.

2. Dosiermechanismus nach Anspruch 1, **dadurch gekennzeichnet, dass** das Stoppelement (300) eine über seinen Umgang und konzentrisch um die Nebendrehachse angeordnete erste Eingriffsstruktur (320), insbesondere Verzahnung, und eines aus erstem Element (100) und zweitem Element (200) eine über seinen Umfang und um die Hauptdrehachse (H) angeordnete zweite Eingriffsstruktur (120; 220), insbesondere Verzahnung, aufweisen, wobei die erste Eingriffsstruktur (320) und die zweite Eingriffsstruktur (120; 220) in einem Eingriffsbereich formschlüssig ineinandergreifen, insbesondere kämmen.

3. Dosiermechanismus nach Anspruch 1 oder 2, **dadurch gekennzeichnet, dass** das Stoppelement (300) eine erste Gleitfläche (330) und das andere aus erstem Element (100) und zweitem Element (200) eine zweite Gleitfläche (130; 230) aufweist, wobei mindestens eine der ersten und zweiten Gleitfläche (330; 130; 230) konzentrisch um die Nebendrehachse (N) angeordnet ist und die erste Gleitfläche (330) und die zweite Gleitfläche (130; 230) während der Drehung des ersten Elements (100) drehend aneinander abgleiten.

4. Dosiermechanismus nach einem der Ansprüche 1 bis 3, **dadurch gekennzeichnet, dass** der Stoppanschlag (110; 210) an einem aus erstem und zweitem Element (100; 200) gebildet ist.

5. Dosiermechanismus nach Anspruch 4, **dadurch gekennzeichnet, dass** der Stoppgegenanschlag (310) quer zur Umfangsrichtung elastisch auslenkbar an dem Stoppelement (300) gebildet ist, wobei eines aus erstem und zweitem Element (100; 200) eine Schaltstruktur (140; 240) aufweist, welche den Stoppgegenanschlag (310) während der Drehung des ersten Elements (100) quer zur Umfangsrichtung elastisch auslenkt, so dass der Stoppgegenanschlag (310) dem Stoppanschlag (110; 210) gegenüberliegt.

6. Dosiermechanismus nach einem der Ansprüche 2 bis 5, **dadurch gekennzeichnet, dass** die zweite Eingriffsstruktur (120) über den Innenumfang des ersten Elements (100) angeordnet ist und dass die erste Eingriffsstruktur (320) über den Außenumfang des Stoppelements (300) angeordnet ist und einen ersten Abschnitt (321) und einen zweiten Abschnitt (322), die entlang der Nebendrehachse (N) zueinander versetzt sind, aufweist, wobei der Stoppgegenanschlag (310) zwischen dem ersten Abschnitt (321) und dem zweiten Abschnitt (322) angeordnet ist, insbesondere an einer elastischen Zunge (323), und insbesondere dass das zweite Element (200) den Stoppanschlag (110) bildet und/oder dass das Stoppelement (300) das zweite Element (200) umgibt.

7. Dosiermechanismus nach einem der Ansprüche 2 bis 5, **dadurch gekennzeichnet, dass** die zweite Eingriffsstruktur (120) über den Innenumfang des ersten Elements (100) angeordnet ist und dass die erste Eingriffsstruktur (320) über den Außenumfang des Stoppelements (300) angeordnet ist, wobei das hülsenförmige Stoppelement (300) entlang der Nebendrehachse (N) einen teilweise oder bevorzugt durchgängigen Schlitz (324) aufweist, wobei eine der den Schlitz (324) einfassenden Wände des Stoppelements (300) den Stoppgegenanschlag (310) bildet, und insbesondere dass das zweite Element (200) den Stoppanschlag (210) bildet und/oder dass das Stoppelement (300) das zweite Element (200) umgibt.

8. Dosiermechanismus nach einem der Ansprüche 2 bis 5, **dadurch gekennzeichnet, dass** die zweite Eingriffsstruktur (220) über den Außenumfang des zweiten Elements (200) angeordnet ist und dass die erste Eingriffsstruktur (320) über den Innenumfang des Stoppelements (300) angeordnet ist, wobei das hülsenförmige Stoppelement (300) entlang der Nebendrehachse (N) einen vollständig oder bevorzugt teilweise durchgängigen Schlitz (325) aufweist, wobei eine der den Schlitz (325) einfassenden Wände des Stoppelements (300) den Stoppgegenanschlag (310) bildet, und insbesondere dass das erste Element (100) den Stoppanschlag (110) bildet und/oder dass das Stoppelement (300) das zweite Element (200) umgibt.

9. Dosiermechanismus nach Anspruch 8, **dadurch gekennzeichnet, dass** das Stoppelement (300) eine an seinem Innenumfang angeordnete, von der ersten Eingriffsstruktur (320) abgesetzte Schaltgegenstruktur (340), insbesondere eine erste Abragung, aufweist und dass das zweite Element (200) eine von der zweiten Eingriffsstruktur abgesetzte Schaltstruktur (240), insbesondere eine zweite Abragung, aufweist, wobei die Schaltstruktur (240) die Schaltgegenstruktur (340) und den Stoppgegenanschlag (310) während der Drehung des ersten Elements (100) quer zur Umfangsrichtung elastisch auslenkt, so dass der Stoppgegenanschlag (310) dem von dem ersten Element (100) gebildeten Stoppanschlag (110) gegenüberliegt.

10. Dosiermechanismus nach einem der Ansprüche 2 bis 5, **dadurch gekennzeichnet, dass** die zweite Eingriffsstruktur (220) über den Innenumfang des zweiten Elements (200) angeordnet ist und dass die erste Eingriffsstruktur (320) über den Außenumfang des Stoppelements (300) angeordnet ist, wobei der Stoppgegenanschlag (310) entlang der Nebendrehachse (N) versetzt zu der ersten Eingriffsstruktur (320) angeordnet ist, insbesondere an einer elastischen Zunge (323), und insbesondere dass das zweite Element (200) den Stoppanschlag (210) bildet und/oder dass das Stoppelement (300) das erste Element (100) umgibt.

11. Dosiermechanismus nach einem der Ansprüche 2 bis 4, **dadurch gekennzeichnet, dass** die zweite Eingriffsstruktur (220) über den Außenumfang des zweiten Elements (200) angeordnet ist und dass die erste Eingriffsstruktur (320) über den Innenumfang des Stoppelements (300) angeordnet ist, wobei die zweite Eingriffsstruktur (220) eine Vielzahl über den Umfang angeordnete Führungsbahnen (221) aufweist, die jeweils ein stirnseitiges Ende (222; 223) aufweisen, wobei das Ende (223) mindestens einer der Führungsbahnen (221) entlang der Hauptdrehachse (H) oder der Nebendrehachse (N) versetzt zu den Enden (222) der anderen Führungsbahnen (221) angeordnet ist, wobei die erste Eingriffsstruktur (320) mindestens ein Eingriffsglied (326) aufweist, wobei das Stoppelement (300) entlang der Hauptdrehachse (H) oder der Nebendrehachse (N) relativ zu dem zweiten Element (200) bewegbar ist, wenn das Eingriffsglied (326) in der Führungsbahn (221) ist, deren Ende (223) versetzt angeordnet ist, wodurch der Stoppgegenanschlag (310) in eine dem Stoppanschlag (110) gegenüberliegende Position verschiebbar ist.

12. Dosiermechanismus nach Anspruch 11, **dadurch gekennzeichnet, dass** die zweite Eingriffsstruktur (220), insbesondere die Führungsbahnen (221), eine Schrägverzahnung ist, und das erste Element (100) und das Stoppelement (300) in einem Reibeingriff sind, wobei das erste Element (100) das Stoppelement (300) aufgrund des Reibeingriffs mitdreht, wenn das Eingriffsglied (326) in der Führungsbahn ist, deren Ende (223) versetzt angeordnet ist, und das erste Element (100) in die erste Drehrichtung gedreht wird, wodurch insbesondere bewirkt wird, dass das Stoppelement (300) zu dem versetzten Ende (223) hin bewegt wird.

13. Dosiermechanismus nach Anspruch 11, **dadurch gekennzeichnet, dass** zwischen dem ersten Element (100) und dem Stoppelement (300) eine vorgespannte Feder (400), insbesondere Druckfeder, angeordnet ist, welche das Stoppelement (300) zu dem versetzten Ende (223) hin verschiebt, wenn das Eingriffsglied (326) in der Führungsbahn ist, deren Ende (223) versetzt ist.

14. Dosiermechanismus nach einem der Ansprüche 2 bis 5, **dadurch gekennzeichnet, dass** die erste Eingriffsstruktur (320) eine Stirnverzahnung und/oder eine Innenverzahnung und die zweite Eingriffsstruktur (120) eine Stirnverzahnung ist, wobei bevorzugt ist, dass das hülsenförmige Stoppelement (300) entlang der Nebendrehachse (N) einen teilweise oder bevorzugt durchgängigen Schlitz (324) aufweist, wobei eine der den Schlitz (324) einfassenden Wände des Stoppelements (300) den Stoppgegenanschlag (310) bildet.

15. Dosiermechanismus nach einem der Ansprüche 1 bis 4, **dadurch gekennzeichnet, dass** das hülsenförmige Stoppelement (300), insbesondere einschließlich seines Stoppgegenanschlags (310), um eine Kippachse, die quer auf die Hauptdrehachse (H) und die Nebendrehachse (N) steht, kippbar ist, wobei eines aus erstem Element (100) und zweitem Element (200) eine Schaltstruktur (140; 240) aufweist, welche das Stoppelement (300) während der Drehung des ersten Elements (100) um die Kippachse kippt, so dass der Stoppgegenanschlag (310) dem Stoppanschlag (140; 240) gegenüberliegt.

## Claims

1. A dosing mechanism for an injection device, comprising:
a) a dosage setting element (2), and a first element (100) which is coupled to the dosage setting element (2) and can be rotated in a first rotational direction about a main rotational axis (H) relative to another, second element (200) by means of a coupling while a dosage is set and which is rotationally fixed relative to the second element (200) while a dosage is delivered, wherein the first element (100) and the second element (200) are coupled via a sleeve-shaped stop element (300) which forms a complementary stop abutment (310); and
b) a stop abutment (110; 210), wherein the complementary stop abutment (310) performs a movement towards a stop position while the first element (100) rotates in the first rotational direction, wherein the complementary stop abutment (310) abuts against the stop abutment (110; 210) in the stop position and prevents the first element (100) from rotating in the first rotational direction relative to the second element (200),
**characterised in that**
c) the sleeve-shaped stop element (300) can be rotated about a secondary rotational axis (N) which is arranged such that it is offset in parallel or is angularly offset with respect to the main rotational axis (H) and which surrounds the first element (100) or the second element (200).

2. The dosing mechanism according to Claim 1, **characterised in that** the stop element (300) has a first engagement structure (320), in particular a toothed gearing, which is arranged over its circumference and concentrically around the secondary rotational axis, and one of the first element (100) and second element (200) has a second engagement structure (120; 220), in particular a toothed gearing, which is arranged over its circumference and around the main rotational axis (H), wherein the first engagement structure (320) and the second engagement structure (120; 220) interlock, in particular mesh, with one another in a positive fit in an engagement region.

3. The dosing mechanism according to Claim 1 or 2, **characterised in that** the stop element (300) has a first sliding area (330), and the other of the first element (100) and second element (200) has a second sliding area (130; 230), wherein at least one of the first and second sliding areas (330; 130; 230) is arranged concentrically around the secondary rotational axis (N), and the first sliding area (330) and the second sliding area (130; 230) rotationally slide off one another while the first element (100) rotates.

4. The dosing mechanism according to any one of Claims 1 to 3, **characterised in that** the stop abutment (110; 210) is formed on one of the first and second elements (100; 200).

5. The dosing mechanism according to Claim 4, **characterised in that** the complementary stop abutment (310) is formed on the stop element (300) such that it can be elastically deflected transversely with respect to the circumferential direction, wherein one of the first and second elements (100; 200) has a switching structure (140; 240) which elastically deflects the complementary stop abutment (310) transversely with respect to the circumferential direction while the first element (100) rotates, such that the complementary stop abutment (310) lies opposite the stop abutment (110; 210).

6. The dosing mechanism according to any one of Claims 2 to 5, **characterised in that** the second engagement structure (120) is arranged over the inner circumference of the first element (100) and **in that** the first engagement structure (320) is arranged over the outer circumference of the stop element (300) and has a first section (321) and a second section (322) which are offset with respect to one another along the secondary rotational axis (N), wherein the complementary stop abutment (310) is arranged between the first section (321) and the second section (322), in particular on an elastic tongue (323), and in particular **in that** the second element (200) forms the stop abutment (110) and/or **in that** the stop element (300) surrounds the second element (200).

7. The dosing mechanism according to any one of Claims 2 to 5, **characterised in that** the second engagement structure (120) is arranged over the inner circumference of the first element (100) and **in that** the first engagement structure (320) is arranged over the outer circumference of the stop element (300), wherein the sleeve-shaped stop element (300) has a partial or preferably continuous slot (324) along the secondary rotational axis (N), wherein one of the walls of the stop element (300) which enclose the slot (324) forms the complementary stop abutment (310), and in particular **in that** the second element (200) forms the stop abutment (210) and/or **in that** the stop element (300) surrounds the second element (200).

8. The dosing mechanism according to any one of Claims 2 to 5, **characterised in that** the second engagement structure (220) is arranged over the outer circumference of the second element (200) and **in that** the first engagement structure (320) is arranged over the inner circumference of the stop element (300), wherein the sleeve-shaped stop element (300) has a completely or preferably partially continuous slot (325) along the secondary rotational axis (N), wherein one of the walls of the stop element (300) which enclose the slot (325) forms the complementary stop abutment (310), and in particular **in that** the first element (100) forms the stop abutment (110) and/or **in that** the stop element (300) surrounds the second element (200).

9. The dosing mechanism according to Claim 8, **characterised in that** the stop element (300) has a complementary switching structure (340), in particular a first projection, which is arranged on its inner circumference and separated from the first engagement structure (320), and **in that** the second element (200) has a switching structure (240), in particular a second projection, which is separated from the second engagement structure, wherein the switching structure (240) elastically deflects the complementary switching structure (340) and the complementary stop abutment (310) transversely with respect to the circumferential direction while the first element (100) rotates, such that the complementary stop abutment (310) lies opposite the stop abutment (110) formed by the first element (100).

10. The dosing mechanism according to any one of Claims 2 to 5, **characterised in that** the second engagement structure (220) is arranged over the inner circumference of the second element (200) and **in that** the first engagement structure (320) is arranged over the outer circumference of the stop element (300), wherein the complementary stop abutment (310) is arranged such that it is offset with respect to the first engagement structure (320) along the secondary rotational axis (N), in particular on an elastic tongue (323), and in particular in that the second element (200) forms the stop abutment (210) and/or **in that** the stop element (300) surrounds the first element (100).

11. The dosing mechanism according to any one of Claims 2 to 4, **characterised in that** the second engagement structure (220) is arranged over the outer circumference of the second element (200) and **in that** the first engagement structure (320) is arranged over the inner circumference of the stop element (300), wherein the second engagement structure (220) has a plurality of guide tracks (221) which are arranged over the circumference and which each have a facing-side end (222; 223), wherein the end (223) of at least one of the guide tracks (221) is arranged such that it is offset with respect to the ends (222) of the other guide tracks (221) along the main rotational axis (H) or the secondary rotational axis (N), wherein the first engagement structure (320) has at least one engagement member (326), wherein the stop element (300) can be moved along the main rotational axis (H) or the secondary rotational axis (N) relative to the second element (200) when the engaging member (326) is in the guide track (221), the end (223) of which is arranged such that it is offset, whereby the complementary stop abutment (310) can be shifted into a position opposite the stop abutment (110).

12. The dosing mechanism according to Claim 11, **characterised in that** the second engagement structure (220), in particular the guide tracks (221), is a helical toothed gearing, and the first element (100) and the stop element (300) are in a frictional engagement, wherein the first element (100) rotates the stop element (300) along with itself due to the frictional engagement when the engagement member (326) is in the guide track, the end (223) of which is arranged such that it is offset, and the first element (100) is rotated in the first rotational direction, which in particular causes the stop element (300) to be moved towards the offset end (223).

13. The dosing mechanism according to Claim 11, **characterised in that** a biased spring (400), in particular a compression spring, which is arranged between the first element (100) and the stop element (300) shifts the stop element (300) towards the offset end (223) when the engaging member (326) is in the guide track, the end (223) of which is offset.

14. The dosing mechanism according to any one of Claims 2 to 5, **characterised in that** the first engagement structure (320) is a facing-side toothed gearing and/or an internally toothed gearing, and the second engagement structure (120) is a facing-side toothed gearing, wherein the sleeve-shaped stop element (300) preferably has a partial or preferably continuous slot (324) along the secondary rotational axis (N), wherein one of the walls of the stop element (300) which enclose the slot (324) forms the complementary stop abutment (310).

15. The dosing mechanism according to any one of Claims 1 to 4, **characterised in that** the sleeve-shaped stop element (300), in particular including its complementary stop abutment (310), can be tilted about a tilting axis which is transverse to the main rotational axis (H) and the secondary rotational axis (N), wherein one of the first element (100) and second element (200) has a switching structure (140; 240) which tilts the stop element (300) about the tilting axis while the first element (100) rotates, such that the complementary stop abutment (310) lies opposite the stop abutment (140; 240).

## Revendications

1. Mécanisme de dosage pour un dispositif d'injection, comprenant :
a) un élément de réglage de dose (2) et un premier élément (100) couplé à ce dernier, qui peut être tourné dans une première direction de rotation autour d'un axe de rotation principal (H) par rapport à un autre deuxième élément (200) au cours d'un réglage de dose au moyen d'un couplage et est solidaire en rotation par rapport au deuxième élément (200) au cours d'une éjection de dose, dans lequel le premier élément (100) et le deuxième élément (200) sont couplés par l'intermédiaire d'un élément d'arrêt (300) en forme de douille, lequel forme une contre-butée d'arrêt (310), et
b) une butée d'arrêt (110 ; 210), dans lequel la contre-butée d'arrêt (310) exécute un déplacement en direction d'une position d'arrêt au cours de la rotation du premier élément (100) dans la première direction de rotation, dans lequel dans la position d'arrêt, la contre-butée d'arrêt (310) vient buter au niveau de la butée d'arrêt (110 ; 210) et empêche la rotation du premier élément (100) par rapport au deuxième élément (200) dans la première direction de rotation,
**caractérisé en ce que**
c) l'élément d'arrêt (300) en forme de douille peut tourner autour d'un axe de rotation secondaire (N), qui est disposé de manière décalée parallèlement ou de manière décalée selon un angle par rapport à l'axe de rotation principal (H) et entoure le premier élément (100) ou le deuxième élément (200).

2. Mécanisme de dosage selon la revendication 1, **caractérisé en ce que** l'élément d'arrêt (300) présente une première structure de prise (320) disposée sur sa périphérie et de manière concentrique autour de l'axe de rotation secondaire, en particulier une denture, et un parmi le premier élément (100) et le deuxième élément (200) présente une deuxième structure de prise (120 ; 220) disposée sur sa périphérie et autour de l'axe de rotation principal (H), en particulier une denture, dans lequel la première structure de prise (320) et la deuxième structure de prise (120 ; 220) s'imbriquent, en particulier s'engagent, l'une dans l'autre par complémentarité de forme dans une zone de prise.

3. Mécanisme de dosage selon la revendication 1 ou 2, **caractérisé en ce que** l'élément d'arrêt (300) présente une première surface de glissement (330) et l'autre parmi le premier élément (100) et le deuxième élément (200) présente une deuxième surface de glissement (130 ; 230), dans lequel au moins une de la première et de la deuxième surface de glissement (330 ; 130 ; 230) est disposée de manière concentrique autour de l'axe de rotation secondaire (N) et la première surface de glissement (330) et la deuxième surface de glissement (130 ; 230) glissent l'une contre l'autre par rotation au cours de la rotation du premier élément (100).

4. Mécanisme de dosage selon l'une quelconque des revendications 1 à 3, **caractérisé en ce que** la butée d'arrêt (110 ; 210) est formée au niveau d'un parmi le premier et le deuxième élément (100 ; 200).

5. Mécanisme de dosage selon la revendication 4, **caractérisé en ce que** la contre-butée d'arrêt (310) est formée au niveau de l'élément d'arrêt (300) de manière à pouvoir être déviée élastiquement de manière transversale par rapport à la direction périphérique, dans lequel un parmi le premier et le deuxième élément (100 ; 200) présente une structure de commutation (140 ; 240), laquelle dévie élastiquement de manière transversale par rapport à la direction périphérique la contre-butée d'arrêt (310) au cours de la rotation du premier élément (100) de sorte que la contre-butée d'arrêt (310) fait face à la butée d'arrêt (110 ; 210).

6. Mécanisme de dosage selon l'une quelconque des revendications 2 à 5, **caractérisé en ce que** la deuxième structure de prise (120) est disposée sur la périphérie intérieure du premier élément (100) et que la première structure de prise (320) est disposée sur la périphérie extérieure de l'élément d'arrêt (300) et présente une première section (321) et une deuxième section (322), qui sont décalées l'une par rapport à l'autre le long de l'axe de rotation secondaire (N), dans lequel la contre-butée d'arrêt (310) est disposée entre la première section (321) et la deuxième section (322), en particulier au niveau d'une languette (323) élastique, et en particulier que le deuxième élément (200) forme la butée d'arrêt (110) et/ou que l'élément d'arrêt (300) entoure le deuxième élément (200).

7. Mécanisme de dosage selon l'une quelconque des revendications 2 à 5, **caractérisé en ce que** la deuxième structure de prise (120) est disposée sur la périphérie intérieure du premier élément (100), et que la première structure de prise (320) est disposée sur la périphérie extérieure de l'élément d'arrêt (300), dans lequel l'élément d'arrêt (300) en forme de douille présente le long de l'axe de rotation secondaire (N) une entaille (324) en partie continue ou de manière préférée continue, dans lequel une des parois, encadrant l'entaille (324), de l'élément d'arrêt (300) forme la contre-butée d'arrêt (310), et en particulier que le deuxième élément (200) forme la butée d'arrêt (210) et/ou que l'élément d'arrêt (300) entoure le deuxième élément (200).

8. Mécanisme de dosage selon l'une quelconque des revendications 2 à 5, **caractérisé en ce que** la deuxième structure de prise (220) est disposée sur la périphérie extérieure du deuxième élément (200), et que la première structure de prise (320) est disposée sur la périphérie intérieure de l'élément d'arrêt (300), dans lequel l'élément d'arrêt (300) en forme de douille présente le long de l'axe de rotation secondaire (N) une entaille (325) totalement continue ou de manière préférée en partie continue, dans lequel une des parois, encadrant l'entaille (325), de l'élément d'arrêt (300) forme la contre-butée d'arrêt (310), et en particulier que le premier élément (100) forme la butée d'arrêt (110) et/ou que l'élément d'arrêt (300) entoure le deuxième élément (200).

9. Mécanisme de dosage selon la revendication 8, **caractérisé en ce que** l'élément d'arrêt (300) présente une contre-structure de commutation (340) disposée au niveau de sa périphérie intérieure, placée en retrait par rapport à la première structure de prise (320), en particulier une première saillie, et que le deuxième élément (200) présente une structure de commutation (240) placée en retrait par rapport à la deuxième structure de prise, en particulier une deuxième saillie, dans lequel la structure de commutation (240) dévie élastiquement de manière transversale par rapport à la direction périphérique la contre-structure de commutation (340) et la contre-butée d'arrêt (310) au cours de la rotation du premier élément (100) de sorte que la contre-butée d'arrêt (310) fait face à la butée d'arrêt (110) formée par le premier élément (100).

10. Mécanisme de dosage selon l'une quelconque des revendications 2 à 5, **caractérisé en ce que** la deuxième structure de prise (220) est disposée sur la périphérie intérieure du deuxième élément (200), et que la première structure de prise (320) est disposée sur la périphérie extérieure de l'élément d'arrêt (300), dans lequel la contre-butée d'arrêt (310) est disposée de manière décalée le long de l'axe de rotation secondaire (N) par rapport à la première structure de prise (320), en particulier au niveau d'une languette (323) élastique, et en particulier que le deuxième élément (200) forme la butée d'arrêt (210) et/ou que l'élément d'arrêt (300) entoure le premier élément (100).

11. Mécanisme de dosage selon l'une quelconque des revendications 2 à 4, **caractérisé en ce que** la deuxième structure de prise (220) est disposée sur la périphérie extérieure du deuxième élément (200), et que la première structure de prise (320) est disposée sur la périphérie intérieure de l'élément d'arrêt (300), dans lequel la deuxième structure de prise (220) présente une pluralité de voies de guidage (221) disposées sur la périphérie, qui présentent respectivement une extrémité côté frontal (222 ; 223), dans lequel l'extrémité (223) d'au moins une des voies de guidage (221) est disposée le long de l'axe de rotation principal (H) ou de l'axe de rotation secondaire (N) de manière décalée par rapport aux extrémités (222) des autres voies de guidage (221), dans lequel la première structure de prise (320) présente au moins un organe de prise (326), dans lequel l'élément d'arrêt (300) peut être déplacé le long de l'axe de rotation principal (H) ou de l'axe de rotation secondaire (N) par rapport au deuxième élément (200) quand l'organe de prise (326) est dans la voie de guidage (221), dont l'extrémité (223) est disposée de manière décalée, ce qui permet de faire coulisser la contre-butée d'arrêt (310) dans une position faisant face à la butée d'arrêt (110).

12. Mécanisme de dosage selon la revendication 11, **caractérisé en ce que** la deuxième structure de prise (220), en particulier les voies de guidage (221), est une denture oblique, et le premier élément (100) et l'élément d'arrêt (300) sont en prise par friction, dans lequel le premier élément (100) entraîne en rotation l'élément d'arrêt (300) en raison de la prise par friction quand l'organe de prise (326) est dans la voie de guidage, dont l'extrémité (223) est disposée de manière décalée, et le premier élément (100) est tourné dans la première direction de rotation ce qui entraîne en particulier le déplacement de l'élément d'arrêt (300) en direction de l'extrémité (223) décalée.

13. Mécanisme de dosage selon la revendication 11, **caractérisé en ce qu'**est disposé entre le premier élément (100) et l'élément d'arrêt (300) un ressort (400) précontraint, en particulier un ressort de pression, lequel fait coulisser l'élément d'arrêt (300) en direction de l'extrémité (223) décalée quand l'organe de prise (326) est dans la voie de guidage, dont l'extrémité (223) est décalée.

14. Mécanisme de dosage selon l'une quelconque des revendications 2 à 5, **caractérisé en ce que** la première structure de prise (320) est une denture frontale et/ou une denture intérieure et la deuxième structure de prise (120) est une denture frontale, dans lequel il est préféré que l'élément d'arrêt (300) en forme de douille présente le long de l'axe de rotation secondaire (N) une entaille (324) en partie continue ou de manière préférée continue, dans lequel une des parois, encadrant l'entaille (324), de l'élément d'arrêt (300) forme la contre-butée d'arrêt (310).

15. Mécanisme de dosage selon l'une quelconque des revendications 1 à 4, **caractérisé en ce que** l'élément d'arrêt (300) en forme de douille, y compris en particulier sa contre-butée d'arrêt (310), peut être basculé autour d'un axe de basculement, qui se situe de manière transversale sur l'axe de rotation principal (H) et l'axe de rotation secondaire (N), dans lequel un parmi le premier élément (100) et le deuxième élément (200) présente une structure de commutation (140 ; 240), laquelle fait basculer autour de l'axe de basculement l'élément d'arrêt (300) au cours de la rotation du premier élément (100) de sorte que la contre-butée d'arrêt (310) fait face à la butée d'arrêt (140 ; 240).
